# EUROPEAN PATENT APPLICATION

(11) **EP 1 571 157 A1**
(43) Date of publication of application: **07.09.2005**
(21) Application number: 04005327.4
(22) Date of filing: 05.03.2004
(51) Int. Cl.: C07K 14/47, C12N 15/11, C12N 15/12, C12N 15/63, A61K 38/17, A61K 48/00, G01N 33/48

(54) **LIPIN1 function**

(71) Applicant: DeveloGen Aktiengesellschaft, 37079 Göttingen (DE)
(72) Inventor: Andag, Uwe, 37115 Duderstadt (DE); Schreiter, Kay, 37081 Göttingen (DE)
(74) Representative: Wichmann, Hendrik

(57) **Abstract**

The present invention addresses the mechanistic role of Lipin in the absence of embryonic development. Accordingly, the present invention relates to novel Lipin1 polypeptides and nucleic acids encoding these polypeptides, which polypeptides are involved in body-weight regulation, energy homeostasis, metabolism, obesity, and diabetes. Furthermore, the invention provides substances modulating the function of the polypeptides of the invention, in particular siRNAs inhibiting Lipin1 function in general and LipinlB2 function in particular and the medical uses of Lipin1 activators and inhibitors.

SEQUENCE LISTING

<110>  DeveloGen AG

<120>  Lipin1 function

<130>  D62619EP

<160>  31

<170>  PatentIn version 3.1

<210>  1
<211>  932
<212>  PRT
<213>  Homo sapiens

<400>  1
   Met Ser Arg Val Gln Thr Met Asn Tyr Val Gly Gln Leu Ala Gly Gln
   1               5                   10                  15
   Val Phe Val Thr Val Lys Glu Leu Tyr Lys Gly Leu Asn Pro Ala Thr
               20                  25                  30
   Leu Ser Gly Cys Ile Asp Ile Ile Val Ile Arg Gln Pro Asn Gly Asn
           35                  40                  45
   Leu Gln Cys Ser Pro Phe His Val Arg Phe Gly Lys Met Gly Val Leu
       50                  55                  60
   Arg Ser Arg Glu Lys Val Val Asp Ile Glu Ile Asn Gly Glu Ser Val
   65                  70                  75                  80
   Asp Leu His Met Lys Leu Gly Asp Asn Gly Glu Ala Phe Phe Val Gln
                   85                  90                  95
   Glu Thr Asp Asn Asp Gln Glu Val Ile Pro Met His Leu Ala Thr Ser
               100                 105                 110
   Pro Ile Leu Ser Glu Gly Ala Ser Arg Met Glu Cys Gln Leu Lys Arg
           115                 120                 125
   Gly Ser Val Asp Arg Met Arg Gly Leu Asp Pro Ser Thr Pro Ala Gln
       130                 135                 140
   Val Ile Ala Pro Ser Glu Thr Pro Ser Ser Ser Ser Val Val Lys Lys
   145                 150                 155                 160
   Arg Arg Lys Arg Arg Arg Lys Ser Gln Leu Asp Ser Leu Lys Arg Asp
                   165                 170                 175
   Asp Asn Met Asn Thr Ser Glu Asp Glu Asp Met Phe Pro Ile Glu Met
               180                 185                 190
   Ser Ser Asp Glu Ala Met Glu Leu Leu Glu Ser Ser Arg Thr Leu Pro
           195                 200                 205
   Asn Asp Ile Pro Pro Phe Gln Asp Asp Ile Pro Glu Glu Asn Leu Ser
       210                 215                 220
   Leu Ala Val Ile Tyr Pro Gln Ser Ala Ser Tyr Pro Asn Ser Asp Arg
   225                 230                 235                 240
   Glu Trp Ser Pro Thr Pro Ser Ser Leu Val Asp Cys Lys Arg Thr Ala
                   245                 250                 255
   Pro His Leu Ala Val Ala Ala Glu Gly Gly Leu Ser Ser Ser Cys Pro
               260                 265                 270
   Pro Gln Ser Ser Leu Phe His Pro Ser Glu Ser Pro Ser Gly Ser Arg
           275                 280                 285
   Pro Ser Thr Pro Lys Ser Asp Ser Glu Leu Val Ser Lys Ser Thr Glu
       290                 295                 300
   Arg Thr Gly Gln Lys Asn Pro Glu Met Leu Trp Leu Trp Gly Glu Leu
   305                 310                 315                 320
   Pro Gln Ala Ala Lys Ser Ser Ser Pro His Lys Met Lys Glu Ser Ser
                   325                 330                 335
   Pro Leu Ser Ser Arg Lys Ile Cys Asp Lys Ser His Phe Gln Ala Ile
               340                 345                 350
   His Ser Glu Ser Ser Asp Thr Phe Ser Asp Gln Ser Pro Thr Leu Val
           355                 360                 365
   Gly Gly Ala Leu Leu Asp Gln Asn Lys Pro Gln Thr Glu Met Gln Phe
       370                 375                 380
   Val Asn Glu Glu Asp Leu Glu Thr Leu Gly Ala Ala Ala Pro Leu Leu
   385                 390                 395                 400
   Pro Met Ile Glu Glu Leu Lys Pro Pro Ser Ala Ser Val Val Gln Thr
   405 410 415
   Ala Asn Lys Thr Asp Ser Pro Ser Arg Lys Arg Asp Lys Arg Ser Arg
   420 425 430
   His Leu Gly Ala Asp Gly Val Tyr Leu Asp Asp Leu Thr Asp Met Asp
   435 440 445
   Pro Glu Val Ala Ala Leu Tyr Phe Pro Lys Asn Gly Asp Pro Ser Gly
   450 455 460
   Leu Ala Lys His Ala Ser Asp Asn Gly Ala Arg Ser Ala Asn Gln Ser
   465 470 475 480
   Pro Gln Ser Val Gly Ser Ser Gly Val Asp Ser Gly Val Glu Ser Thr
   485 490 495
   Ser Asp Gly Leu Arg Asp Leu Pro Ser Ile Ala Ile Ser Leu Cys Gly
   500 505 510
   Gly Leu Ser Asp His Arg Glu Ile Thr Lys Asp Ala Phe Leu Glu Gln
   515 520 525
   Ala Val Ser Tyr Gln Gln Phe Val Asp Asn Pro Ala Ile Ile Asp Asp
   530 535 540
   Pro Asn Leu Val Val Lys Ile Gly Ser Lys Tyr Tyr Asn Trp Thr Thr
   545 550 555 560
   Ala Ala Pro Leu Leu Leu Ala Met Gln Ala Phe Gln Lys Pro Leu Pro
   565 570 575
   Lys Ala Thr Val Glu Ser Ile Met Arg Asp Lys Met Pro Lys Lys Gly
   580 585 590
   Gly Arg Trp Trp Phe Ser Trp Arg Gly Arg Asn Thr Thr Ile Lys Glu
   595 600 605
   Glu Ser Lys Pro Glu Gln Cys Leu Ala Gly Lys Ala His Ser Thr Gly
   610 615 620
   Glu Gln Pro Pro Gln Leu Ser Leu Ala Thr Arg Val Lys His Glu Ser
   625 630 635 640
   Ser Ser Ser Asp Glu Glu Arg Ala Ala Ala Lys Pro Ser Asn Ala Gly
   645 650 655
   His Leu Pro Leu Leu Pro Asn Val Ser Tyr Lys Lys Thr Leu Arg Leu
   660 665 670
   Thr Ser Glu Gln Leu Lys Ser Leu Lys Leu Lys Asn Gly Pro Asn Asp
           675                 680                 685
   Val Val Phe Ser Val Thr Thr Gln Tyr Gln Gly Thr Cys Arg Cys Glu
       690                 695                 700
   Gly Thr Ile Tyr Leu Trp Asn Trp Asp Asp Lys Val Ile Ile Ser Asp
   705                 710                 715                 720
   Ile Asp Gly Thr Ile Thr Arg Ser Asp Thr Leu Gly His Ile Leu Pro
                   725                 730                 735
   Thr Leu Gly Lys Asp Trp Thr His Gln Gly Ile Ala Lys Leu Tyr His
               740                 745                 750
   Lys Val Ser Gln Asn Gly Tyr Lys Phe Leu Tyr Cys Ser Ala Arg Ala
           755                 760                 765
   Ile Gly Met Ala Asp Met Thr Arg Gly Tyr Leu His Trp Val Asn Glu
       770                 775                 780
   Arg Gly Thr Val Leu Pro Gln Gly Pro Leu Leu Leu Ser Pro Ser Ser
   785                 790                 795                 800
   Leu Phe Ser Ala Leu His Arg Glu Val Ile Glu Lys Lys Pro Glu Lys
                   805                 810                 815
   Phe Lys Val Gln Cys Leu Thr Asp Ile Lys Asn Leu Phe Phe Pro Asn
               820                 825                 830
   Thr Glu Pro Phe Tyr Ala Ala Phe Gly Asn Arg Pro Ala Asp Val Tyr
           835                 840                 845
   Ser Tyr Lys Gln Val Gly Val Ser Leu Asn Arg Ile Phe Thr Val Asn
       850                 855                 860
   Pro Lys Gly Glu Leu Val Gln Glu His Ala Lys Thr Asn Ile Ser Ser
   865                 870                 875                 880
   Tyr Val Arg Leu Cys Glu Val Val Asp His Val Phe Pro Leu Leu Lys
                   885                 890                 895
   Arg Ser His Ser Ser Asp Phe Pro Cys Ser Asp Thr Phe Ser Asn Phe
               900                 905                 910
   Thr Phe Trp Arg Glu Pro Leu Pro Pro Phe Glu Asn Gln Asp Ile His
           915                 920                 925
   Ser Ala Ser Ala
       930

<210> 2
<211> 19
<212> RNA
<213> artificial sequence

<220>
<223> core of siRNA against human Lipin1, sense strand
<400> 2
ugauucagaa uuggucagc 19

<210> 3
<211> 19
<212> RNA
<213> artificial sequence

<220>
<223> core of siRNA against human Lipin1, antisense strand
<400> 3
gcugaccaau ucugaauca 19

<210> 4
<211> 19
<212> DNA
<213> artificial sequence

<220>
<223> DNA complementary to sense strand of siRNA against human Lipin1
<400> 4
gctgaccaat tctgaatca 19

<210> 5
<211> 19
<212> DNA
<213> artificial sequence

<220>
<223> DNA complementary to antisense strand of siRNA against human Lip
in1

<400> 5
tgattcagaa ttggtcagc 19

<210> 6

<211> 2897

<212> DNA

<213> Homo sapiens

<400> 6
   gtaactctga agcgtgagct gccagaattc aaggccacct gcttaacaga agacaactag 60
   aaacctcatc catgagcaga gtgcagacca tgaattacgt ggggcagtta gccggccagg 120
   tgtttgtcac cgtgaaggag ctctacaagg ggctgaatcc cgccacactc tcagggtgca 180
   ttgacatcat tgtcatccgc cagcccaatg gaaacctcca atgctcccct ttccacgtcc 240
   gctttgggaa gatgggggtc ctgcgctccc gagagaaagt ggttgacata gaaatcaatg 300
   gggaatctgt ggatttgcat atgaaattgg gagataatgg agaagcattt tttgttcaag 360
   aaacagataa tgatcaggaa gttatcccta tgcacctggc cacctccccc atcctgtcag 420
   aaggagcttc gagaatggaa tgccagctga aaaggggctc tgtggacagg atgagaggcc 480
   tggaccccag cacgccagcc caagtgatcg ctcccagcga gacgccgtca agcagctctg 540
   tagtaaagaa gagaagaaaa aggaggagaa agtcacagct ggacagcctg aagagagatg 600
   acaacatgaa cacatctgag gatgaggaca tgttccccat cgagatgagc tcggatgagg 660
   ccatggagct gctggagagc agcagaactc ttcctaatga tatacctcca ttccaagatg 720
   atattcctga ggaaaacctc tccctggctg tgatttaccc tcagtcagcc tcatacccta 780
   attcggatag agagtggtca cccactccca gtagcctggt agattgcaaa aggactgccc 840
   ctcatcttgc agttgcggcc gagggaggtc tgtctagttc ttgccctcca cagtcttccc 900
   tgttccatcc ttcggaaagt ccttccggtt cccgaccttc aacacctaaa agtgattcag 960
   aattggtcag caagtccacg gaaaggacag ggcagaagaa cccagaaatg ctttggctgt 1020
   ggggagagct gccgcaggct gctaagtctt cttctccaca caagatgaaa gagtccagcc 1080
   cattgagcag tagaaaaatt tgtgataaaa gtcactttca ggccattcac agcgaatctt 1140
   cagacacttt tagtgaccaa tcgccaactc tggtcggtgg ggcacttttg gaccagaaca 1200
   agcctcagac agaaatgcag tttgtgaatg aagaagacct ggagacctta ggagcagcag 1260
   cgccactctt gcccatgatc gaggagctca aacccccctc tgccagtgta gtccagacag 1320
   caaacaagac ggattctcct tccaggaaaa gagataaacg aagccgacat cttggtgctg 1380
   acggcgtcta cttggatgac ctcacagaca tggatcctga agtggcggcc ctgtattttc 1440
   ccaaaaacgg agatccttcc ggactcgcaa aacatgcaag cgacaacgga gcccggtcag 1500
   ccaaccagtc cccgcagtcg gtgggcagct cgggcgtgga cagtggcgtg gagagcacct 1560
   cggacgggct gagggacctc ccttccatcg ccatctccct ctgcgggggc ctcagcgacc 1620
   accgggagat cacgaaagat gcattcctgg agcaagctgt gtcatatcaa cagtttgtgg 1680
   acaaccccgc tattatcgat gaccccaatc tcgtggtaaa gattgggagt aaatattata 1740
   actggacaac agcagcaccc ctcctcctgg caatgcaggc cttccagaaa cctttgccaa 1800
   aggccactgt ggaatctatc atgagggata aaatgcccaa aaagggagga agatggtggt 1860
   tttcatggag gggaagaaac accacaatca aggaggaaag taagccagag cagtgcttgg 1920
   ctggcaaggc ccatagcacc ggagagcaac cgccgcagct cagcttggcc accagggtaa 1980
   agcatgaatc atcctccagt gatgaggagc gcgcagctgc caagccatca aacgcaggcc 2040
   acctccctct tctgcctaat gtcagctaca agaagactct ccggctgact tccgagcagc 2100
   ttaaaagctt gaagttgaag aatggcccca acgacgtggt tttcagtgtc accacgcagt 2160
   accaaggcac gtgccgctgt gagggcacca tctatctgtg gaactgggat gataaagtca 2220
   tcatttctga tattgatggg acaattacca gatcagatac tcttggccac attttgccca 2280
   cccttgggaa ggattggacc catcagggca tcgctaagct gtaccataaa gtgagccaga 2340
   atggatataa atttctctac tgttctgccc gtgccatcgg gatggcggac atgacgcggg 2400
   gctacctgca ctgggtcaac gagaggggca cggtgctgcc ccaggggccc ctgctgctga 2460
   gtcccagcag cctcttctct gccctgcaca gagaagtgat tgaaaagaag ccagaaaagt 2520
   ttaaagtcca gtgtttgaca gacatcaaaa acctgttttt ccccaacaca gaaccctttt 2580
   atgctgcttt tggaaaccga ccagctgatg tgtattcata caagcaagta ggagtgtctt 2640
   tgaatagaat atttaccgtc aaccctaaag gagagctggt acaggaacat gcaaagacca 2700
   acatctcttc gtatgtgaga ctctgtgaag tagtcgacca cgttttcccg ttgctgaaaa 2760
   gaagccattc ttcagacttt ccctgttcgg ataccttcag taacttcacc ttttggagag 2820
   agccactgcc accttttgaa aaccaggaca ttcattctgc ctcagcgtaa aatgtcccaa 2880
   gcagcctctt gccagca 2897

<210> 7
<211> 37
<212> DNA
<213> artificial sequence

<220>
<223> Primer sense strand
<400> 7
ctagtctaga gaattcctcg gtgcagacca tgaatta 37

<210> 8
<211> 23

<212>  DNA

<213>  artificial sequence

<220>

<223>  Primer antisense strand

<400>  8
ccacttcagg atccatgtct gtg                                                23

<210>  9

<211>  21

<212>  DNA

<213>  artificial sequence

<220>

<223>  Primer sense strand

<400>  9
gtctacttgg atgacctcac a                                                  21

<210>  10

<211>  31

<212>  DNA

<213>  artificial sequence

<220>

<223>  Primer antisense strand

<400>  10
accgctcgag tgctggcaag aggctgcttg g                                       31

<210>  11

<211>  29

<212>  DNA

<213>  artificial sequence

<220>

<223>  Primer sense strand

<400>  11
ggggtaccgt aactctgaag cgtgagctg                                          29

<210> 12

<211> 23

<212> DNA

<213> artificial sequence

<220>

<223> Primer antisense strand

<400> 12
ccacttcagg atccatgtct gtg 23

<210> 13

<211> 16

<212> DNA

<213> artificial sequence

<220>

<223> Primer sense strand

<400> 13
ccggagagca accgcc 16

<210> 14

<211> 16

<212> DNA

<213> artificial sequence

<220>

<223> Primer antisense strand

<400> 14
gctgcgcgct cctcat 16

<210> 15

<211> 30

<212> DNA

<213> artificial sequence

<220>

<223> Probe

<400> 15
accagggtaa agcatgaatc atcctccagt 30

<210> 16

<211> 19

<212> DNA

<213> artificial sequence

<220>

<223> Primer sense strand

<400> 16
ggtcacccac tcccagtcc 19

<210> 17

<211> 24

<212> DNA

<213> artificial sequence

<220>

<223> Primer antisense strand

<400> 17
tgctgaccaa ttctgaatca cttt 24

<210> 18

<211> 19

<212> DNA

<213> artificial sequence

<220>

<223> siRNA

<400> 18
tgattcagaa ttggtcagc 19

<210> 19

<211> 23

<212> DNA

<213> artificial sequence

<220>

<223>  Probe

<400>  19
tccggttccc gaccttcaac acc                                              23

<210>  20

<211>  20

<212>  DNA

<213>  artificial sequence

<220>

<223>  Primer sense strand

<400>  20
cccggtagat tgcaaaagga                                                  20

<210>  21

<211>  23

<212>  DNA

<213>  artificial sequence

<220>

<223>  Primer antisense strand

<400>  21
agaactagac agacctccct cgg                                              23

<210>  22

<211>  22

<212>  DNA

<213>  artificial sequence

<220>

<223>  Probe

<400>  22
tgcccctcat cttgcagttg cg                                               22

<210>  23

<211>  27

<212>  DNA

<213>  artificial sequence

<220>

<223>  Primer sense strand

<400>  23
atggaagaca ctgtctgtac catagtg                                          27

<210>  24

<211>  21

<212>  DNA

<213>  artificial sequence

<220>

<223>  Primer antisense strand

<400>  24
cgagaagctc tgccacagat g                                                21

<210>  25

<211>  25

<212>  DNA

<213>  artificial sequence

<220>

<223>  Probe

<400>  25
cctgggtaca cagatgagcg accca                                            25

<210>  26

<211>  18

<212>  DNA

<213>  artificial sequence

<220>

<223>  Primer sense strand

<400>  26
cacgtgcgtt ttggcaag                                                    18

<210>  27

<211> 24

<212> DNA

<213> artificial sequence

<220>

<223> Primer antisense strand

<400> 27
agctcaatgt ctaccacctt ctcc 24

<210> 28

<211> 18

<212> DNA

<213> artificial sequence

<220>

<223> Probe

<400> 28
tgggcgtcct gcggtcgc 18

<210> 29

<211> 19

<212> DNA

<213> artificial sequence

<220>

<223> siRNA

<400> 29
gatcaggaag ttatcccta 19

<210> 30

<211> 19

<212> DNA

<213> artificial sequence

<220>

<223> siRNA

<400> 30

tcatcttgca gttgcggcc 19

<210> 31

<211> 19

<212> DNA

<213> artificial sequence

<220>

<223> siRNA

<400> 31
gaggtctgtc tagttcttg 19

## Description

### BACKGROUND OF THE INVENTION

There are several metabolic diseases of human and animal metabolism, e.g., obesity and severe weight loss, that relate to energy imbalance where caloric intake versus energy expenditure is imbalanced. Obesity is one of the most prevalent metabolic disorders in the world. It is still a poorly understood human disease that becomes more and more relevant for western society. Obesity is defined as a body weight more than 20% in excess of the ideal body weight, frequently resulting in a significant impairment of health. Obesity may be measured by body mass index, an indicator of adiposity or fatness. Further parameters for defining obesity are waist circumferences, skin-fold thickness and bioimpedance. Obesity is associated with an increased risk for cardiovascular disease, hypertension, diabetes, hyperlipidemia and an increased mortality rate. Besides severe risks of illness, individuals suffering from obesity are often isolated socially.

Obesity is influenced by genetic, metabolic, biochemical, psychological, and behavioral factors, and can be caused by different reasons such as non-insulin dependent diabetes, increase in triglycerides, increase in carbohydrate-bound energy and low energy expenditure. As such, it is a complex disorder that must be addressed on several fronts to achieve lasting positive clinical outcome. Since obesity is not to be considered as a single disorder but as a heterogeneous group of conditions with (potential) multiple causes, it is also characterized by elevated fasting plasma insulin and an exaggerated insulin response to oral glucose intake (Koltermann O.G., (1980) *J Clin Invest,* 65:1272-1284). A clear involvement of obesity in type-2 diabetes mellitus can be confirmed (Kopelman P.G., (2000) *Nature,* 404:635-643).

The molecular factors regulating food intake and body weight balance are incompletely understood. Even if several candidate genes have been described which are supposed to influence the homeostatic system(s) that regulate body mass/weight, like leptin or the peroxisome proliferator-activated receptor-gamma co-activator, the distinct molecular mechanisms and/or molecules influencing obesity or body weight/body mass regulations are not known. In addition, several single-gene mutations resulting in obesity have been described in mice, implicating genetic factors in the etiology of obesity (Friedman J.M. and Leibel R.L., (1992), *Cell,* 69: 217-220). In the obese (ob) mouse a single gene mutation (obese) results in profound obesity, which is accompanied by diabetes (Friedman J.M. *et. al.,* (1991) *Genomics,* 11:1054-1062).

The Lipin1 (*lpin1)* gene was originally cloned from mice harboring an autosomal recessive mutation, the *fatty liver dystrophy* (*fld*) (Langner C.A. *et al.,* (1989) *J Biol Chem,* 264:7994-8003; Peterfy M. *et al.,* (2001) *Nat Genet,* 27:121-124). Homozygous *fld*/*fld* mice exhibit several metabolic abnormalities, e.g. reduced plasma leptin levels, profound glucose intolerance, insulin resistance, and significantly higher disposition to arteriosclerosis (Reue K. *et al.,* (2000) *J Lipid Res* 41: 1067-1076). *fld* mice develop a liver steatosis after birth, which spontaneously returns to normal by the suckling-to-weaning transition at 14-18 days of age. Although this reversion coincides with the change from a lipid-rich diet to a carbohydrate-rich diet, it has been demonstrated that reversion occurs even if mice are prevented from weaning by prolonged suckling. This rules out the possibility that reversion of the fatty liver results from the cessation of suckling or of exposure to triglycerides or other components of mother's milk. Furthermore, the fatty liver is not reinduced by feeding adult *fld* mice a triglyceride-enriched diet (Rehnmark S. *et al.,* (1998) *J Lipid Res* 39:2209-2217).

*fld* mice exhibit 80% reduction in adipose tissue mass. Epididymal fat tissue of 1-month-old, *fld* mice consists mainly of immature adipocytes.

Two *lpin1* mutant alleles are described in the prior art. The *fld* mutant allele leads to a loss of *lpin1* mRNA expression, whereas the *fld*^{*2J*} allele represents a single point mutation (G84R) in a highly conserved region of the protein. Both mutant mice exhibit identical phenotypes (Peterfy *et al., supra*).

In mice *lpin1* shows highest expression in adipose tissue, skeletal muscle and testis. *fld* males are infertile. Lipin1 contains nuclear localization signal within the N-terminal part and a Lipin1-GFP chimera protein localizes predominantly inside the nucleus (Peterfy *et al., supra*).

Lipin1 was identified as a target of mTOR and is phosphorylated on serine and threonine residues in response to insulin treatment of cells (Huffman T.A. *et al.,* (2002*) Proc Natl Acad Sci USA,* 99:1047-1052).

Cao H. and Hegele R.A. (2002; *J Hum Genet,* 47:370-372) identified single-nucleotide polymorphisms in the human *LPIN1* gene. As all of these are silent the authors suggest that *LPIN1* mutations are not commonly seen in patients with lipodystrophy.

Lipin1 seems to be a key regulator during development in mice. The loss of gene function leads to primary phenotypic effects in adipose tissue and testis but no overt phenotypes are seen in other tissues although the protein has been shown to be present in for example *thymus,* spleen, liver and lung. Since the observed phenotype in the *fld* mice indicates a role for Lipin1 function already during embryonic development (a phenotype is already manifest shortly after birth), it cannot be assessed what further physiological significance Lipin1 function has in the body of an animal; for example, the role of Lipin1 function in an adult animal remains obscure. This kind of insight, however, would be necessary for predicting the value of Lipin as a drug target for the treatment of specific disorders and diseases in humans and animals.

In mouse and human, Lipin2 and Lipin3 have been identified. Potentially these proteins are responsible for a redundant function of Lipin1 in some tissues but not in others. Both proteins share two conserved regions of high similarity with Lipin1. No significant homology to other known proteins has been described so far. Tomita M. et al. (2002; *Arch Toxicol,* 76:530-537) reported that Lipin2 is upregulated in the lungs of rats under oxidative stress. They find this upregulation in Clara cells (secretory cells) and macrophages in lung tissue.

The present invention provides insight into the mechanistic function of Lipin1 in general and of a new form of Lipin1, Lipin1B2, in particular, allowing for the first time the rational application of modulators, that is inhibitors and activators, of Lipin1 function as medicaments for the treatment of several diseases.

Therefore, the technical problem underlying the present invention was to provide new means and methods for modulating/effecting (pathological) metabolic conditions influencing body-weight regulation and/or energy homeostatic circuits.

The solution to said technical problem is achieved by providing the embodiments characterized in the claims.

### SUMMARY OF THE INVENTION

The present invention addresses the mechanistic role of Lipin1 in the absence of embryonic development. Accordingly, the present invention relates to novel Lipin1 polypeptides and nucleic acids encoding these polypeptides, which polypeptides are involved in body-weight regulation, energy homeostasis, metabolism, obesity, and diabetes. Furthermore, the invention provides substances modulating the function of the polypeptides of the invention, in particular siRNAs inhibiting Lipin1 function in general and Lipin1B2 function in particular and the medical uses of Lipin1 activators and inhibitors.

### DEFINITIONS

As used herein, an "siRNA" (small interfering RNA) is a double-stranded RNA interfering with gene expression of the mRNA against which it is targeted.

Recently it has been found that short, double-stranded RNAs potently and specifically inhibit expression of a mRNA with a complementary exonic sequence. This phenomenon has been termed RNA interference (RNAi). These double-stranded RNAs have been termed siRNAs, for small interfering RNAs, they have a paired region of at least 18, preferably at least 19 nucleotides (this paired, double-stranded region targets them against an mRNA with a complementary exonic sequence), and have a length of 20 nucleotides to 25 nucleotides, preferably of 21 nucleotides to 23 nucleotides (Elbashir *et al.,* (2001) *Nature,* 411, 428-429). These siRNAs can be synthesized chemically by methods well known in the art and are commercially available (see e.g. suppliers given in Elbashir *et al.* above and also in Elbashir *et al.,* (2002) *Methods,* 26, 199-213) or by T7 transcription off a suitable DNA template (see Yu *et al.* (2002) *PNAS,* 99, 6047-6052). They can be delivered to a wide range of cell types, in which inhibition of gene expression of a certain gene is desired, by, e.g., synthesizing the two RNA strands, annealing them and transfecting them (see Elbashir *et al.* and Yu *et al.,* above). SiRNAs have shown to function in gene downregulation also *in vivo* (Zender *et al.,* (2003) *Proc Natl Acad Sci USA,* 100(13):7787-7802). Detailed protocols for the application of siRNAs are described in Elbashir *et al.,* (2002) *Methods,* 26, 199-213.

Another way of effecting RNA interference is to transfect a plasmid that leads to the cellular production of siRNAs or siRNA like hairpin RNAs (shRNAs), which are also functional in RNAi, as will be described further below. This can be done by plasmids which carry both, the sense and the antisense strand of the siRNA under the control of a mammalian, preferably human or mouse, Pol III promoter, like a U6 or H1 promoter, which leads to the transcription of both strands in a transfected cell, some of which anneal to form functional double-stranded siRNAs within the transfected cell (Miyagishi and Taira (2002) *Nature Biotech,* 19, 497-500). Alternatively, the RNA species transcribed from the Pol III promoter can be siRNA-like hairpin RNAs (shRNAs) which consist of a 19-base pair siRNA stem with the two strands joined by a structured loop and a 3' overhang at the end of the antisense strand (see Paul *et al.* (2002) *Nature Biotech,* 19, 505-508). Alternatively the RNA species transcribed from the Pol IIIpromoter can be transcribed as a small temporal RNA (stRNA), hairpin precursors of about 70 nucleotides, which can be processed into active siRNAs or shRNAs within the cell in which they are transcribed (see Paddison *et al.* (2002) *Genes and Development,* 16, 948-958). All these methods based on transfection of plasmids have in common that they lead to the cellular production of siRNAs or shRNAs, which leads to the inhibition of the gene with an exonic region complementary to the at least 18 nucleotide long base paired region of the siRNA or shRNA. It is clear that also future ways to affect the cellular presence of siRNAs or shRNAs will lead to the desired effect of inhibiting lipin function and are within the scope of the invention.

A "polypeptide" as used herein is a molecule comprising more than 30 and in particular more than 35, 40, 45 or even more than 50 amino acids, but less than 10,000, in particular less than 9,000, 8,000, 7,000, 6,000, 5,000, 4,000, 3,000 or 2,000, most preferably less than 1,500 amino acids. Polypeptides are usually linear amino acid polymers, wherein the individual amino acids are linked to one another via peptide bonds. Also, polypeptides which contain a low percentage, e.g. less than 5%, 3% or even only up to 1% of modified or non-natural amino acids, are encompassed. Polypeptides can be further modified by chemical modification, e.g. by phosphorylation of serine, theonine or thyrosine residues, or by glycosylation, e.g. of asparagins or of serine residues.

As used herein, the term "recombinant expression cassette" is meant to include any type of genetic construct containing a nucleic acid coding for a gene product in which part or all of the nucleic acid sequence is capable of being transcribed. The transcript may be translated into a protein, but it need not be, as, for example, in the case of expression cassettes encoding siRNAs, shRNAs or stRNAs it is the transcript itself which is the active species or which is transformed to the active species. Thus, the term "expression" includes transcription, and in certain embodiments the term includes transcription and translation. In order for the expression cassette to effect expression of a transcript, the polynucleotide to be transcribed will be under the transcriptional control of a promoter. A "promoter" refers to a DNA sequence recognized by the transcriptional machinery of the host cell that is required to initiate the specific transcription of a gene. In order for the expression cassette to effect expression of a siRNA, shRNA or stRNA of the invention, the promoter is preferably a Pol III promoter, like a H1 or U6 promoter.

The phrase "operatively linked" as used herein means that the polynucleotide to be expressed is under transcriptional control of a promoter, i.e. the promoter is in the correct location and in the correct orientation in relation to the polynucleotide to be transcribed to control RNA polymerase initiation and transcription of the polynucleotide.

The term "pharmaceutically acceptable carrier" as used herein refers to substances and compositions that do not produce an adverse, allergic or other untoward reaction when administered to an animal or a human in particular. As used herein, a "pharmaceutically acceptable carrier" includes any and all solvents, dispersion media, coatings, antibacterial and antifungal agents, isotonic and absorption delaying agents, and the like. In those cases where pharmaceutical compositions of the invention comprising the siRNAs of the invention are contemplated, it is preferred that care is taken to prepare these compositions in an overall RNAse-free way. There are known precautionary measures, like wearing gloves during the preparation of the compositions or like pre-treating the containers used during the preparation of the pharmaceutical with chemicals in order to destroy RNAse activity on the surfaces of the containers. This is preferably carried out before preparation of the pharmaceutical.

An "adipocyte" as used herein is a specialized cell that stores energy as triglyceride in the form of fat droplets, and releases energy as free fatty acids and glycerol during periods of energy need. In addition to releasing free fatty acids (FFA) which can function as fuels or signals, adipocytes can modify steroid hormones leading to production of estrogen and glucocorticoids, and release an increasing number of hormones, hormone precursors, hemostatic regulators, or cytokines (Flier J.S., 2004, *Cell* 116: 337-350). Adipocytes can be identified by way of molecular markers like adipocyte protein aP2 (aP2), glucose transporter 4 (GLUT4), hormone-sensitive lipase (HSL) and CCAAT-enhancer binding protein-alpha (C/EBPalpha).

A "preadipocyte" as used herein is an adipocyte precursor cell. Preadipocytes can be cultured as growing precursor cells or differentiated with medium supplemented with adipogenic and lipogenic hormones. Preadipocyte can be identified by way of molecular markers like Pre-adipocyte factor-1 (named Prefl in mouse) and cyclooxygenase-2.

A "pancreas cell" as used herein is one of six major types of cells present in the pancreas. These are duct cells, exocrine acinar cells, and the four principal types of islet cells. The functional unit of the endocrine pancreas is the islet of Langerhans which are scattered throughout is the exocrine portion of the pancreas and are composed of four cell types: (alpha-, beta-, gamma- and PP-cells, reviewed in Slack, *Development* 121 (1995), 1569-1580. Beta-cells produce insulin, represent the majority of the endocrine cells and form the core of the islets, while alpha-cells secrete glucagon and are located in the periphery. Gamma-cells and PP-cells are less numerous and respectively secrete somatostatin and a pancreatic polypeptide.

A "primary cell" as used herein is a cell or cell line taken directly from a living organism (e.g. biopsy material), which is not immortalized.

An "insulin sensitizer" as used herein is a substance that increases insulin-stimulated glucose uptake into insulin-sensitive tissues like muscle and adipose tissue and therefore can be used as treatment for insulin resistance in type 2 diabetes mellitus.

An "insulin secretagogue" as used herein includes, for example, Meglitinide (PRANDIN), Repaglinide (PRANDIN), and Nateglinide (Starlix), Sulfonylurea derivates: e.g. carbutamid (NADISAN), Tolbutamid (Rastinon, Artison, Tolbutamid ratiopharm), Glimidin (REDUL), Tolazamid (NORGLYCIN), Gliclazid (DIAMICRON), Glipizid (GLIBENESE), and functionally equivalent compounds. Sulfonylureas stimulate insulin release by binding to the sulfonylurea receptor on beta cells.

"Thiazolidinediones" as used herein are a class of oral medicine for type 2 diabetes that helps insulin take glucose from the blood into the cells for energy by making cells more sensitive to insulin. TZDs arepotent, selective agonists of the peroxisome proliferator-activated receptor gamma (PPAR-γ), a nuclear receptor expressed in tissues that are targets for insulin action, including skeletal muscle, liver, and adipose tissue. PPAR-γ plays a role in the production, transport, and utilization of glucose and in the regulation of fatty acid metabolism. The principal function of TZDs in the treatment of diabetes mellitus type 2 is sensitisation of target tissues to the effects of endogenous and exogenous insulin. Thus, thiazolidinedione (TZD) compounds are widely used as oral hypoglycemic agents. For example, glitazones like rosiglitazone (Avandia), pioglitazone (Actos), and troglitazone (Rezulin) are currently available TZDs.

"Biguanides" as used herein are a class of oral anti-diabetics, generated by fusion of 2 molecules guanidine (or/and derivates) under release of ammonia. They are a class of oral medicine used to treat type 2 diabetes that lowers blood glucose by reducing the amount of glucose produced by the liver and by helping the body respond better to insulin. Metformin (GLUCOPHAGE®) is an example of a currently available oral biguanide.

"Alpha glucosidase inhibitors" as used herein are a class of oral medicine for type 2 diabetes that blocks glucosidases like from the class(es) EC 3.2.1.20 and/or E.C.3.2.1.3. that digest starches in food, in particular from the class EC 3.2.1.20. The result is a slower and lower rise in blood glucose throughout the day, especially right after meals. Acarbose (Precose; Glucobay®) and miglitol (GLYSET) are examples of currently available alpha-glucosidase inhibitors.

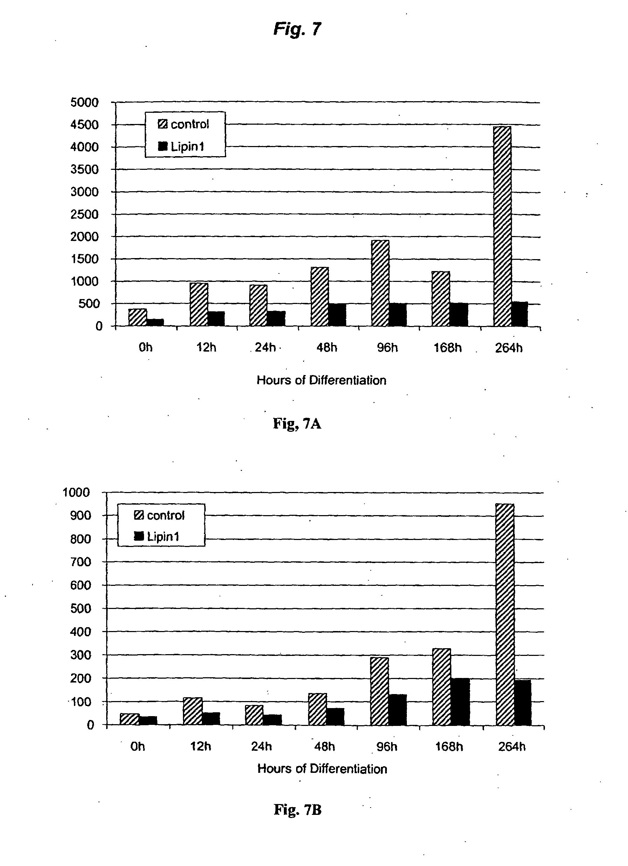

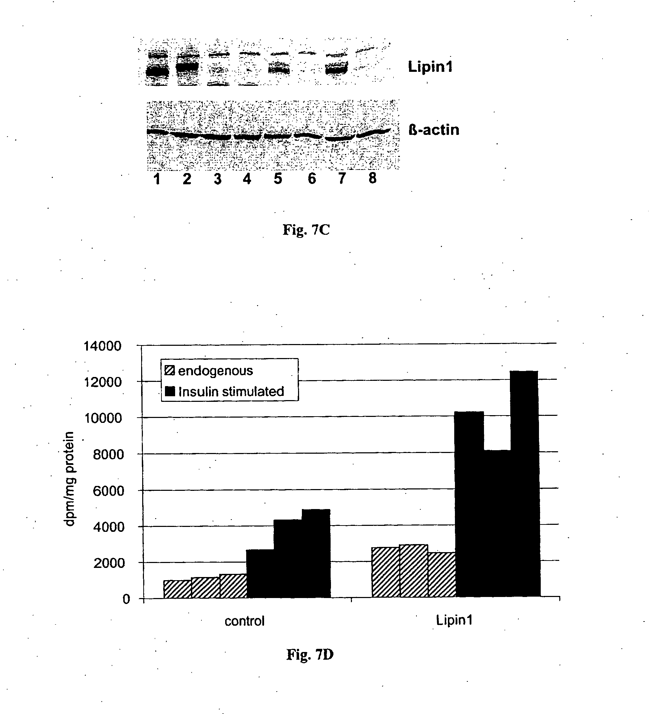

An "inhibitor of lipin function" as used herein is a substance which decreases the relative amount of lipin polypeptide present in a cell treated within the range of 1 pM - 100 µM of the inhibitor, in particular when present at 100nM, by 30% and more, preferably by 50% and more. Alternatively, an "inhibitor of lipin function" binds to the lipin polypeptide with a Kd -ranging from 1 pM to 1 µM, preferably from 10 pM to 100 nM, more preferably from 100 pM to 10 nM, and increases insulin-stimulated glycogen uptake to a level of 125% or more, more preferably from 125% to 300%, when present at a concentration corresponding to 3 x its Kd for lipin, in comparison to the glycogen uptake level of insulin-stimulated control cells without the inhibitor (see example shown in Figure 7C).

An "activator of lipin function" as used herein is a substance, which leads to a delay in adipocyte differentiation when applied to SGBS-cells in concentrations within a range from 1 pM - 100 µM, preferably when present at a concentration of 100 nM.

A "direct target of PKB" as used herein is a polypeptide directly phosphorylated by PKB and includes, for example, mTOR, Phosphofructokinase 2, BAD, FKHR-L1, GSK3, PP2A, IRS1, p21 and PKCξ/δ (Lawlor and Alessi, 2001, *J Cell Sci.* 114:2903-2910).

A "substrate of mTOR" as used herein is a polypeptide directly phosphorylated by mTOR and includes, for example, p70 S6 kinase (p70 S6K) and PHAS-I (Lawrence *et al., Curr Top Microbiol Immunol.* 2004;279:199-213).

A "substrate of GSK3" as used herein is a polypeptide directly phosphorylated by GSK3 (glycogen synthase kinase 3) and includes, for example, Kinesin light chains (KLCs) (Morfini *G. et al.,* 2002, *EMBO J.* 21:281-293), Glycogen synthase, eIF2B and ATPcitrate lyase (Bevan P., 2001, *J Cell Sci.* 114:1429-1430).

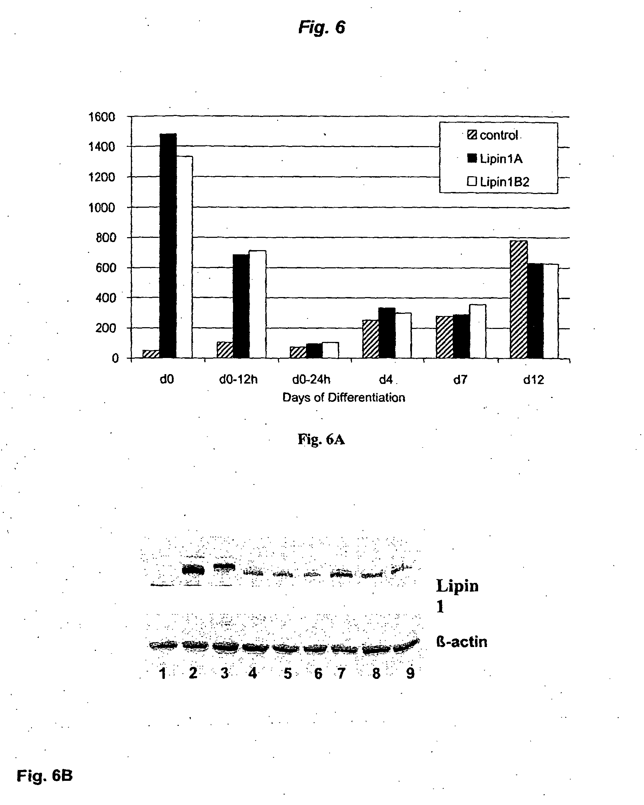

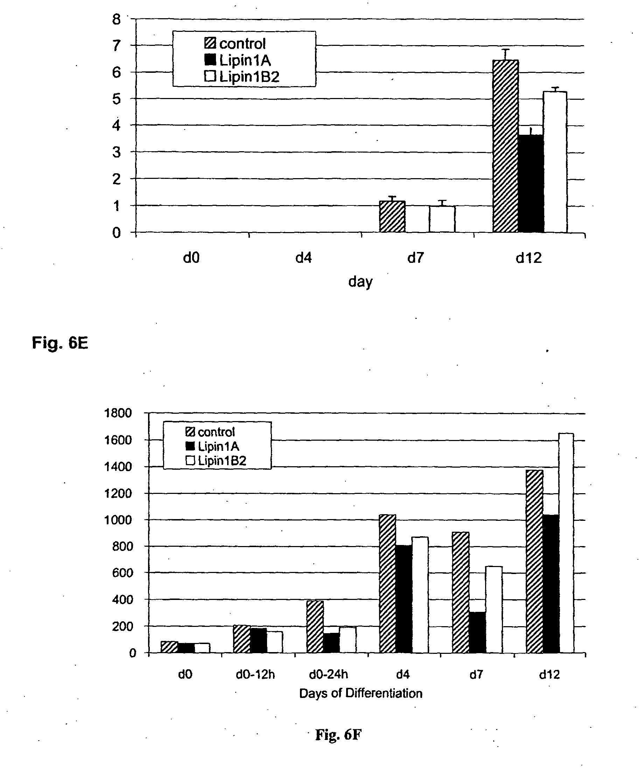

A "delay in adipocyte differentiation" as used herein means an increase of at least 25% in the time needed for an SGBS-cell to differentiate into an adipocyte in an experiment as shown in Figure 6F. The "time needed for an SGBS-cell to differentiate into an adipocyte" is defined as the time point at which expression of PPAR gamma reaches 50% of the day 12 volume in untreated control SGBS-cells.

### DETAILED DESCRIPTION OF THE PREFERRED EMBODIMENTS

The present invention relates to an siRNA molecule comprising the double-stranded region

5' UGAUUCAGAAUUGGUCAGC 3' (SEQ ID NO:2)

3' ACUAAGUCUUAACCAGUCG 5' (SEQ ID NO:3).

It has surprisingly been found that an siRNA molecule of the invention is an effective inhibitor of Lipin1 expression. Such an inhibitor up to now has not been described in the prior art and is useful in further studies on Lipin1 function, as will be described in the examples. Contrary to the *lpd* mouse mutants, the inhibitors of the invention now allow Lipin1 function to be analysed at any stage of development, thereby revealing Lipin1 functions at later time points in development, for example in the adult, which are unamenable to study these in the mutant mouse systems due to an earlier requirement for Lipin1 function during development.

It is preferred that the siRNA molecule of the invention has 2 or 3 nucleotides added to the 3' end of SEQ ID NO:2, preferably U residues. It is also preferred that the siRNA molecule of the invention has 3 or 4 nucleotides added to the 3' end of SEQ ID NO:3, preferably U residues. Most preferably, the 3' overlays are added both to SEQ ID NO:2 and SEQ ID NO:3. Such overhanging 3' ends will further increase the efficiency of the siRNA molecule of the invention, thereby leading to an even higher degree of inhibition of Lipin1 gene expression.

In another aspect, this invention relates to a polypeptide comprising, and in particular consisting of, the amino acid sequence of SEQ ID NO:1, and to a nucleic acid molecule which comprises a nucleotide sequence which encodes a polypeptide of the invention, particularly a nucleic acid molecule as part of an expression construct. SEQ ID NO:1 depicts a novel and unexpected form of the Lipin1 polypeptide, which differs at several positions from the polypeptid Lipin1B, in particular at the extreme N terminus. For the purposes of this invention, the new polypeptide has therefore been named Lipin1B2 and its significance in metabolism and adipocyte development has been demonstrated by the experiments shown in the examples.

In a preferred embodiment, the nucleic acid is a DNA or an RNA. In an RNA U residues take the place of T residues in a DNA.

In another aspect, the invention relates to a recombinant expression cassette comprising
a) the nucleic acid encoding the polypeptide of the invention operatively linked to at least one regulatory sequence for expression of the polypeptide of the invention; or
b) a nucleic acid comprising a nucleotide sequence SEQ ID NO:4 and a nucleotide sequence complementary to SEQ ID NO:5, operatively linked to at least one regulatory sequence for transcription of an RNA molecule comprising the nucleotide sequences SEQ ID NO:2 and SEQ ID NO:3; or
c) a nucleic acid comprising a nucleotide sequence SEQ ID NO:4, operatively linked to at least one first regulatory sequence for transcription of an RNA comprising the nucleotide sequence SEQ ID NO:2, in combination with a nucleic acid comprising a nucleotide sequence SEQ ID NO:5, operatively linked to at least one second regulatory sequence, for transcription of an RNA comprising the nucleotide sequence SEQ ED NO:3.

The recombinant expression cassette of the invention preferably also further comprises expression control sequences which are operatively linked to said nucleic acid molecules including either a polypeptide of the invention or (part of) an siRNA or an shRNA or an stRNA of the invention. The expression control sequences are chosen so that they allow expression of the polypeptide of the invention or of the siRNA or shRNA or stRNA of the invention in a host. A recombinant expression cassette may be a recombinant expression vector. For example, a nucleic acid sequence encoding a polypeptide of the invention can be isolated and cloned into an expression vector and the vector can then be transformed into a suitable host cell for expression of the polypeptide of the invention. Such a vector can be a plasmid, a phageamid or a cosmid. For example, a nucleic acid molecule of the invention can be cloned in a suitable fashion into prokaryotic or eukaryotic expression vectors, preferably into eukaryotic expression vectors and more preferably into expression vectors allowing expression in a mammalian and in particular in a human cell (*Molecular Cloning: A Laboratory Manual,* 3^{rd} edition, Eds. Sambrook *et al.,* CSHL Press 2001). Such expression vectors typically comprise at least one promoter and can also comprise a signal for translation initiation of the reading frame encoding the polypeptide of the invention and - in the case of prokaryotic expression vectors - a signal for translation termination, while in the case of eukaryotic expression vectors the expression cassette preferably comprises expression signals for transcriptional termination and polyadenylation. Examples of suitable eukaryotic expression vectors are those described by Ficcerone *et al., "Generation of recombinant Baculovirus DNA in E. coli using Baculovirus shuttle vector"* (1997) volume 13, U. Reischt Eds. (Totoba NJ: Humana Press Inc.) for the expression in insect cells via baculovirus vectors, and for expression in mammalian cells, e.g. the SV40 or CMV vectors, which are commonly known and commercially available, or the sindbisvirus expression system (Schlesinger (1993), *Trans Bio Technol,* 11(1):18-22) or an adenovirus expression system (Hea *et al.* (1998), *Proc Natl Acad Sci, USA* 95:2509-2514), or a Semliki Forest Virus-based expression system (Smerdou and Liljestrom, 1999) or a lentivirus-based expression system (Ehrengruber, M.U., and Lundstrom, K. (2000). Alphavirus-mediated gene transfer in neurons. In *Current Protocols in Neuroscience,* eds. Crawley, J., Gerfen, C., McKay, R., Rogawski, M., Sibley, D. and Skolnik, P. John Wiley & Sons, New York, Unit 4.21; Smerdou and Liljestrom (1999) *J Virol,* 73(2):1092-8; Naldini *et al.* (1966) *Science,* 1966 Apr. 12;272(5259):263-7). The molecular biological methods for the production of these expression vectors are well known to the skilled person, as well as the methods for transfecting host cells and culturing such transfected host cells. In a preferred embodiment the above-mentioned expression control sequences specify induced expression of the polypeptide of the invention, that is induced transcription of the messenger RNA encoding the polypeptide of the invention upon addition or withdrawal of an external signal, such as a small chemical like tetracycline or a hormone like Ecdysone, but the extracellular signal can also be an increase or decrease in temperature or ionizing radiation. Also, inducible expression can be brought about by inducible translation initiation of the messenger RNA or a system in which mRNA stability is controlled in an inducible fashion. Examples of expression control sequences allowing induction of polypeptide production are reviewed in the following publications: the TET-off/TET-on system, suitable for both cell cultures and transgenic animals, for example described by Gossen *et al.* (1995) *Science,* Jun 23;268(5218):1766-9; adapted for use in transgenic animals by, for example, Kistner *et al.* (1996) *Proc Natl Acad Sci USA,* 93:10933-10938, but also the expression control system based on Cre-recombinase based methods, predominantly for use in transgenic animals, for example described by Lakso *et al.* (1992) *Proc Natl Acad Sci USA,* Jul 15;89(14):6232-6. A further inducible expression system, for use in both cell culture and transgenic animals is based on the insect hormone Ecdysone, for example described by *Hoppe et al.* (2000) *Mol Ther,* 1:159-164. Another inducible expression system is the GAL4 system, which has been successfully applied with mice (Ornitz *et al.* (1991) *Proc Natl Acad Sci USA,* Feb 1 ;88(3):698-702), zebrafish (*Scheer et al.* (1999) *Mech Dev,* Feb;80(2):153-8) and Drosophila (Brand and Perrimon (1993) *Development,* Jun;118(2):401-15), and allows conditional expression at 26-29 degrees, or also a Rapamycin based conditional expression system (Pollock *et al.* (2000) *Proc Natl Acad Sci USA,* Nov 21;97(24):13221-6). A temperature-sensitive expression system is based on a Sindbis virus expression cassette (Boorsma *et al.* (2000) *Nat Biotechnol,* Apr;18(4):429-32) and predominantly suitable for controlled expression in cell culture systems.

In a preferred embodiment the recombinant expression cassette of the invention is capable of leading to the generation of an siRNA molecule or shRNA molecule of the invention, for example upon transfection into a host cell. In the case of such expression cassettes, expression cassettes are preferred that lead to the cellular production of siRNAs or shRNAs (siRNA-like hairpin RNAs) or to the cellular production of stRNAS (small temporal RNAs) which can then be intracellularly processed, for example by DICER, to functional siRNAs or shRNAs. This can be done by employing expression cassettes which carry both the sense and the antisense strand of the siRNA under control of a mammalian Pol III promoter, preferably a human, mouse or rat Pol III promoter, like, for example, the U6 or H1 promoter. These promoters then direct transcription of both strands in the transfected cells and some of those strands anneal to form functional double-stranded siRNAs within the transfected cell (Miyagishi and Taira (2002) *Nature Biotech,* 19,497-500). Thus, in a preferred embodiment the invention relates to a recombinant expression cassette comprising a nucleic acid comprising a nucleotide sequence SEQ ID NO:4, operatively linked to at least one first regulatory sequence for transcription of an RNA comprising the nucleotide sequence SEQ ID NO:2, for example a nucleic acid comprising a nucleotide sequence SEQ ID NO:4 downstream of a U6 or H1 promoter, which arrangement gives rise to transcription of an RNA comprising the nucleotide sequence SEQ ID NO:2. Also present on this recombinant expression cassette can be, and preferably is, a nucleic acid comprising a nucleotide sequence SEQ ID NO:5, operatively linked to at least one second regulatory sequence (for example a nucleic acid comprising a nucleotide sequence SEQ ID NO:5 downstream of a U6 or H1 promoter), which will give rise to transcription of an RNA comprising the nucleotide sequence SEQ ID NO:3. Thus, preferably, such recombinant expression cassettes comprise the nucleic acid molecule coding for the sense strand of the siRNA molecule under control of a first promoter, preferably a promoter for polymerase III, like the U6 or H1 promoter, and they further comprise a nucleic acid sequence encoding the antisense strand of the siRNA molecule of the invention under control of a second promoter, preferably wherein the second promoter is also a Pol III promoter, like the U6 or H1 promoter. Preferably, the first and the second promoters are independent from one another, more preferably they are both Pol III promoters, most preferably they are both either the H1 promoter or the U6 promoter or one is the H1 promoter and the other is the U6 promoter.

In another preferred embodiment, the recombinant expression cassette comprises a nucleic acid comprising a nucleotide sequence SEQ ID NO:4 and a nucleic acid comprising a nucleotide sequence SEQ ID NO:5, operatively linked to at least one regulatory sequence, for transcription of an RNA comprising the nucleotide sequences SEQ ID:2 and SEQ ID:3. For example, the transcribed RNA can be a stRNA which can then be processed intracellularly, e.g. by DICER, to an active siRNA molecule or shRNA molecule of the invention. Preferably, the at least one regulatory sequence for transcription is a promoter, more preferably a polymerase III promoter, and most preferably an H1 or U6 promoter. Preferably, the RNA comprising the nucleotide sequences SEQ ID NO:2 and SEQ ID NO:3 has a length from 40-200 ribonucleotides, more preferably 50-150 ribonucleotides, and most preferably from 60-120 ribonucleotides (stRNA). It is preferred that the resulting transcribed RNA is capable of forming a hairpin structure *in vivo* and is capable of being processed, for example by DICER, into siRNA molecules or shRNA molecules of the invention. Alternatively, the transcribed RNA molecule can also be a short hairpin RNA (shRNA) molecule with siRNA activity. Preferably such shRNA molecules have a length of from 40-60, more preferably of from 44-50 ribonucleotides.

In cell culture or *in vivo* siRNAs may also be generated by transcription of the individual RNA strands from different expression cassettes, e.g. from different expression vectors. Therefore, in another aspect, the present invention relates to a combination of two expression cassettes, particularly two expression vectors, wherein a first expression cassette, e.g. an expression vector, comprises a nucleic acid comprising a nucleotide sequence SEQ ID NO:4, operatively linked to at least one regulatory sequence, for example located downstream of a promoter, like a pol III promoter, in particular an H1 or U6 promoter, for transcription of an RNA comprising the nucleotide sequence SEQ ID NO:2, and wherein a second expression cassette, e.g. an expression vector, comprises a nucleic acid comprising a nucleotide sequence SEQ ID NO:5, operatively linked to at least one regulatory sequence, for example located downstream of a promoter, particularly a pol III promoter, most preferably a U6 or H1 promoter, for transcription of an RNA comprising the nucleotide sequence SEQ ID NO:3. It is preferred that the transcribed RNAs have a length of from 19-25 nucleotides, preferably, 20-24 nucleotides, and more preferably 21-23 nucleotides. It is further preferred that the transcribed RNAs end in 2, 3, or 4 U residues at the 3' end. After transcription of the two RNA strands in a cell, some of the strands anneal with one another to form active siRNAs, interfering with Lipin1 expression. In another aspect the invention relates to a host cell comprising a polypeptide of the invention and/or a nucleic acid of the invention and/or an expression cassette of the invention and/or an siRNA or shRNA of the invention and/or a combination of two expression cassettes of the invention. Such a host cell can be a mammalian, non-human cell inside or outside of the animal body or a human cell outside of the human body. But it can also be an insect cell, like a *Drosophila* cell, in culture or in the context of a transgenic insect, like a transgenic *Drosophila.* Preferred host cells are mammalian, and particularly human or mouse cells or rat cells, more preferably a human, mouse or rat adipocyte, preadipocyte, muscle cell or pancreas cell, even more preferably a cell selected from the group consisting of SGBS cells, 293FT cells, 3T3-L1 cells, CCL-136 cells, primary preadipocytes, primary skeletal muscle cells and pancreatic β-cells, most preferably wherein said cells are human cells. These cells can be made capable of expressing the polypeptide of the invention by introducing the nucleic acid encoding the polypeptide of the invention or the expression cassette of the invention encoding the polypeptide of the invention, for example by transfection with such a nucleic acid or expression cassette. Other means of introducing the nucleic acid and/or the expression cassette of the invention are "gene gun" approaches, mRNA transfer, viral infection, microinjection or liposomal nucleic acid transfer, to name but a few. In the case of a host cell comprising an siRNA of the invention or an expression cassette or a combination of expression cassettes of the invention, which lead to generation of siRNAs of the invention or shRNAs of the invention, the means of introducing the siRNA or shRNA or expression cassette or combination of expression cassettes of the invention to the above-mentioned cells are again the above-mentioned nucleic acid transfer methods, preferably liposomal nucleic acid transfer. Since expression of the siRNA or shRNA of the invention shows strong and potent interference with Lipin1 expression, it is, in those cases where the significance of Lipin1 function during certain stages of development are to be examined, preferred that in such host cells the expression of the siRNA or shRNA of the invention is initially very low or off, for example during the generation of a stably transformed cell line, and only for experimental purposes and after establishment of such a stably transformed cell line the expression of the siRNA or shRNA of the invention is turned on by addition of a suitable stimulus, like e.g. a hormone like ecdysone or a small chemical like the antibiotic tetracyclin. Such inducible systems for siRNA and/or shRNA expression have, for example, been described by Wetering et al., *EMBO Rep.* (2003) 4:609-15; Czaudema et al., *Nucleic Acids Res.* (2003) 31:e127; and Sioud, *Trends Pharmacol Sci.)(*2004) 25:22-8, which are hereby incorporated by reference.

In another aspect, the invention relates to a transgenic animal which comprises a host cell of the invention. Particularly preferred are transgenic non-human mammals, like transgenic rodents, e.g. transgenic mouse or transgenic rat. For the generation of transgenic mice with suitable cell- or tissue-specific promoters, reference is made to Hogan D. *et al.* (1994) "Production of transgenic mice" in *Manipulating the Mouse Embryo: A Laboratory Manual* - Hogan D., Konstantini F., Lacey E. Eds., Cold Spring Harbor Laboratory Press, Cold Spring, NY, pp. 217-252. As mentioned above, expression of an siRNA or shRNA or stRNAs of the invention can be made inducible. With regard to inducible siRNA expression systems or shRNA expression systems or stRNA expression systems in transgenic mice, reference is made to Halbanese C. *et al.,* "Recent advances in inducible expression in transgenic mice" (2002) *Semin Cell Biol* 13:129-41. Wetering et al., *EMBO Rep.* (2003) 4:609-15;; Wiznerowicz & Trono; *J Virol.* (2003) 77:8957-61 have described inducible systems for effecting RNAi in the mouse and are hereby incorporated by reference. Methods for the generation of transgenic mice are known in the art. Moreover, under the control of tissue-specific promoters, expression of siRNAs or shRNAs of the invention could be targeted to specific tissues in mice and others, for example to adipocytes (CCAAT-enhancer binding protein-alpha (C/EBPalpha) -promoter), preadipocytes (Pre-adipocyte factor-1 (Pref-1)-promoter), skeletal muscle cells (Myoblast determination protein (MyoD)-promoter), pancreatic β-cells (Pancreas/duodenum homeobox-1 (Pdx-1)-promoter). For RNAi experiments to be carried out in the mouse, an siRNA or shRNA is to be used which corresponds to SEQ ID NO:2 and SEQ ID NO:3, but which is adapted to the mouse mRNA sequence.

As evidenced in the examples, the polypeptide of the invention, and particularly the siRNAs and shRNAs of the invention or the expression cassettes of the invention or the combination of two expression cassettes of the invention are of use in medicine, particularly for treating, preventing and/or delaying a disorder or a disease selected from the group consisting of metabolic syndrome, diabetes mellitus, obesity, stroke, arteriosclerosis or cancer.

In another aspect, the invention therefore relates to a pharmaceutical composition comprising a polypeptide of the invention or an siRNA or shRNA of the invention or an expression cassette of the invention or a combination of two expression cassettes of the invention, and a pharmaceutically acceptable carrier. When the clinical application of the polypeptide of the invention or the siRNA or shRNA of the invention or the expression construct of the invention or the combination of two expression constructs of the invention is contemplated it will be appropriate to prepare a pharmaceutical composition appropriate for the intended application. Generally this will entail preparing a pharmaceutical composition that is essentially free of pyrogens, as well as any other impurities that could be harmful to humans or animals. One will generally desire to employ appropriate salts and buffers to render the molecules of the invention stable and allow for their uptake by target cells.

Aqueous pharmaceutical compositions of the present invention comprise an effective amount of the polypeptide of the invention or the siRNA or the shRNA of the invention or the expression cassette of the invention or the combination of two expression cassettes of the invention, dissolved or dispersed in a pharmaceutically acceptable carrier or aqueous medium. Such pharmaceutical compositions do not produce an adverse, allergic or untoward reaction when administered to an animal or to a human. The pharmaceutical composition of the invention may further include solvents and combinations thereof, dispersion media, coatings, antibacterial and/or antifungal agents, isotonic and absorption delaying agents, and the like. The use of such media and agents for pharmaceutical active substances is well known in the art. The pharmaceutical compositions of the invention may be administered via any common route so long as the target tissue is accessible via that route. This includes oral, nasal, buccal, rectal, vaginal or topical administration. Alternatively, administration will be orthotopic, intradermal, intraocular, subcutaneous, intramuscular, intraperitoneal or intravenous injection. For such modes of administration the pharmaceutical compositions may include physiologically acceptable carriers, buffers or other excipients. In a preferred embodiment the pharmaceutical composition of the invention comprises a siRNA or shRNA of the invention and is essentially free of RNAses. Preferably it is a solution of siRNAs or shRNAs of the invention in physiologic saline solution, which can preferably be administered via intravenous injection. Preferably, the amount of siRNAs or shRNAs to be administered is in the range from 0.1 - 1 nM (see also Zender et al, PNAS 2003, 100(13):7797-7802).

In a preferred embodiment, the pharmaceutical composition of the invention further comprises an insulin sensitizer or insulin secretagogue, and in particular further comprises a thiazolidinedion, for example glitazones like rosiglitazone (Avandia), pioglitazone (Actos), and troglitazone (Rezulin); an insulin secretagogue like, for example, Meglitinide (PRANDIN), Repaglinide (PRANDIN), and Nateglinide (Starlix), Sulfonylurea derivates: e.g. carbutamid (NADISAN), Tolbutamid (Rastinon, Artison, Tolbutamid ratiopharm), Glimidin (REDUL), Tolazamid (NORGLYCIN), Gliclazid (DIAMICRON), Glipizid (GLIBENESE), and functionally equivalent compounds; a biguanide, like, for example, Metformin (GLUCOPHAGE®); or an alpha-glucosidase inhibitor, like, for example Acarbose (Precose; Glucobay®) and miglitol (GLYSET).

In another aspect, the invention relates to the use of a modulator of Lipin1 function in the manufacture of a pharmaceutical for the treatment and/or prevention and/or the delaying of the onset of a disorder or a disease selected from the group consisting of metabolic syndrome, diabetes mellitus, obesity, stroke, arteriosclerosis, or cancer. As shown in the experimental section, inhibition of Lipin1 function leads to an increased glucose uptake and increased glycogen synthesis by adipocytes. Thus, *in vivo* an inhibitor of Lipin1 function is expected to increase insulin sensitivity of a cell, thereby lowering blood-glucose levels. Thus, in a preferred embodiment, the invention relates to the use of an inhibitor of lipin1 function, like an siRNA or shRNA targeted against the Lipin1A mRNA and/or the Lipin1B mRNA, in particular the siRNA or shRNA of the invention, or an inhibitory polypeptide or peptide or an inhibitory small chemical molecule, in the manufacture of a pharmaceutical for the treatment and/or prevention and/or the delaying of the onset of a disorder or a disease selected from the group consisting of diabetes mellitus, arteriosclerosis, muscular dystrophy, heart attack, and stroke. Without wishing to be bound by any theory, it is believed that Lipin1, in addition to its influence on glucose and/or glycogen metabolism in adipocytes, has an inhibitory activity on the anti-apoptotic polypeptide PKB/Akt. Inhibiting Lipin1 function should therefore activate PKB, which activation will help cell-survival after conditions like stroke or heart attack.

In another preferred embodiment, the invention relates to the use of an activator of lipin function, like an activatory small chemical molecule or and activatory polypeptide or peptide, for the treatment and/or prevention and/or the delaying of the onset of a disorder or disease selected from the group consisting of obesity and cancer. Without wishing to be bound by any theory, activating Lipin1 function is believed to inhibit PKB function, which inhibition will be beneficial in the treatment of conditions where PKB/Akt is functioning like an oncogene, for example in ovarian, breast and pancreatic cancer (Cheng et al. *Proc Natl Acad Sci U S A.* (1992), 89:9267-71; Cheng et al. *Proc Natl Acad Sci U S A.* (1996), 93:3636-41; Nakatani et al*J Biol Chem.* (1999)274:21528-32). An additional function of Lipin1 activation can be a delay of adipocyte development, providing a possible rationale for the role of Lipin1 activators in the treatment of obesity.

In another preferred embodiment, the medicament is to be administered to a mammal, preferably a human being, a cat, a dog, a mouse, or a rat, more preferably by the above-mentioned routes of administration.

In another aspect, the invention relates to the use of an siRNA or an shRNA or an stRNA directed against a nucleotide sequence on the mouse, rat or human, and preferably human, Lipin1-mRNA, preferably the Lipin1B1- and/or Lipin1B2-mRNA, either within 50 nucleotides (nt) upstream of the sequence 5' UGAUUCAGAAUUGGUCAGC 3' starting from the 5' U or 50nt downstream of said sequence starting from the 3' C, preferably within 20nt upstream or 20nt downstream of said sequence, most preferably within 15nt upstream or 15nt downstream of said sequence, for reducing expression of Lipin, preferably Lipin1, more preferably Lipin1B1 and Lipin1B2, *in vivo,* in the case of a non-human mammal, or *ex vivo,* and preferably *in vitro.* Contrary to initial expectations about RNAi, it has proven to be difficult to define those areas of a messenger RNA which are indeed accessible to RNA interference. The present invention provides such an accessible area on the Lipinl-, and in particular the Lipin1B1- and Lipin1B2-mRNA, which is, without wishing to be bound by any theory, possibly free of mRNA-binding proteins and therefore accessible to RNA-treatment. This situation may well be conserved among mammalian species. Other siRNAs or shRNAs targeted against the mRNA of Lipin1, particularly the mRNA of Lipin1B1 and Lipin1B2, in this particular area are predicted to show an improved inhibitory effect with regard to decreasing stability of the Lipin-mRNA in comparison to siRNAs or shRNAs targeted against other regions of the Lipin-mRNA. The present invention therefore enables a skilled person to produce such siRNAs or shRNAs which are capable of reducing the expression of Lipin1. In another aspect, the invention therefore relates to a method of producing an siRNA or shRNA capable of reducing expression of Lipin1, preferably Lipin1B1 and Lipin1B2, comprising the steps of a) providing an siRNA or shRNA directed against a nucleotide sequence on the Lipin-1-mRNA, preferably the Lipin1B1- and/or the Lipin1B2-mRNA, either within 50 nucleotides (nt) upstream of the sequence 5' UGAUUCAGAAUUGGUCAGC 3' starting from the 5' U or 50 nucleotides downstream of said sequence starting from the 3' C, preferably within 30 nt upstream or 30 nt downstream of said sequence, more preferably within 20 nt upstream or 20 nt downstream of said sequence, most preferably within 15 nt upstream or 15 nt downstream of said sequence, and b) optionally testing whether said siRNA or shRNA reduces expression of Lipin1, preferably Lipin1B1 or Lipin1B2. Testing whether the siRNA or shRNA reduces expression of Lipin1 can be carried out like in the example shown in Figures 7A, 7B and 7C, namely by comparing the amount of Lipin-mRNA in cells treated with the siRNA of the invention or shRNA of the invention in comparison to untreated control cells, or by comparing the amount of Lipin1, proteins in treated cells in comparison to the amount present in untreated control cells, as for example shown in the experiment shown in Figure 7C.

The present invention describes the phenotype of increased Lipin1 activity as well as of decreased Lipin1 activity in adipocytes in cell culture. Surprisingly, it has been found that an inhibitor of lipin function, preferably an inhibitor of Lipin1 function, more preferably an inhibitor of Lipin1B1 and/or Lipin1B2 function leads to a detectable increase in glucose uptake, glycogen formation and/or an increase in phosphorylation of polypeptides that are direct PKB-targets or targets downstream of PKB, like substrates of mTOR or GSK3. In another aspect, the invention therefore relates to a method of identifying an inhibitor of lipin function, preferably Lipin1 function, more preferably Lipin1B and7or Lipin1B2 function, comprising the step of a) contacting a mammalian, preferably a human, mouse or rat, adipocyte, a preadipocyte, a muscle cell or a pancreas cell, preferably a cell selected from the group consisting of SGBS cells, 293FT cells, 3T3-L1 cells, CCL-136 cells, primary preadipocytes, primary skeletal muscle cells and pancreatic β-cells, with a test compound, for example a small chemical molecule or a mix of chemical molecules as present in a complex library of chemical molecules, and b) detecting an increase in glucose uptake, glycogen formation or an increase in phosphorylation of polypeptides that are direct PKB-targets, for example, mTOR, Phosphofructokinase 2, BAD, FKHR-L1, GSK3, PP2A, IRS1, p21 and PKCξ/δ, or downstream targets (i.e. targets downstream of PKB), like substrates of mTOR, for example p70 S6 kinase (p70 S6K), PHAS-I, or lipin, or substrates of GSK3 like, for example, Kinesin light chains (KLCs), Glycogen synthase, eIF2B and ATPcitrate lyase.

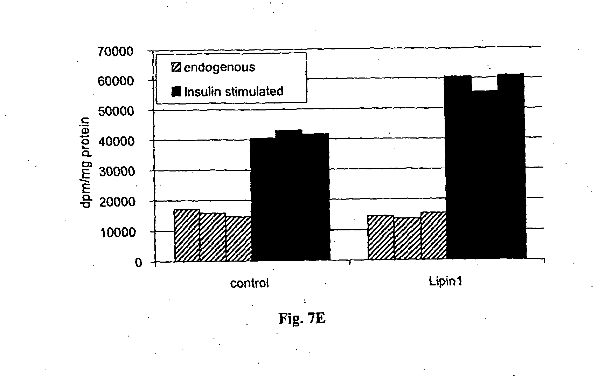

Glycogen synthesis can be examined as shown in the example presented in Figure 7D, glucose uptake can be determined as shown in the example presented in Figure 7E. Preferably, an increase in glycogen synthesis, glucose uptake or phosphorylation is determined by way of comparison with a control sample of cells which has not been treated with the compound to be tested. The suitable time range for detecting the phenotypic changes after contacting with the test compound are from 0 days to 21 days, preferably from 1 hour to 14 days, more preferably from 2 hours to 12 days. It is preferred that phenotypic changes in phosphorylation patterns are examined at earlier time points after contacting with the test compound, for example from 5 minutes to 48 hours, more preferably from 15 minutes to 24 hours, most preferably from 30 minutes to 12 hours, while preferably phenotypic changes in glucose uptake and/or glycogen synthesis may be carried out at later time points, like from day 3 to day 21, more preferably from day 4 to day 16, most preferably from day 5 to day 14. Preadipocytes can also be allowed to differentiate to adipocytes and then the test compound can be administered, for example on days 1-3, 3-5, 5-7, 7-9, 9-11, 11-13, 13-16, 16-21 after the begin of adipocyte differentiation the test compound may be administered, and then the above-mentioned phenotypic assays may be carried out. In a preferred embodiment, preadipocytes have been allowed to differentiate to adipocytes for 7-21 days, more preferably for 9 to 18 days, and then the test compound is contacted with the cells for 10min to 1 day, more preferably for 30min to 12 hours, and then the above-mentioned phenotypic assays are carried out.

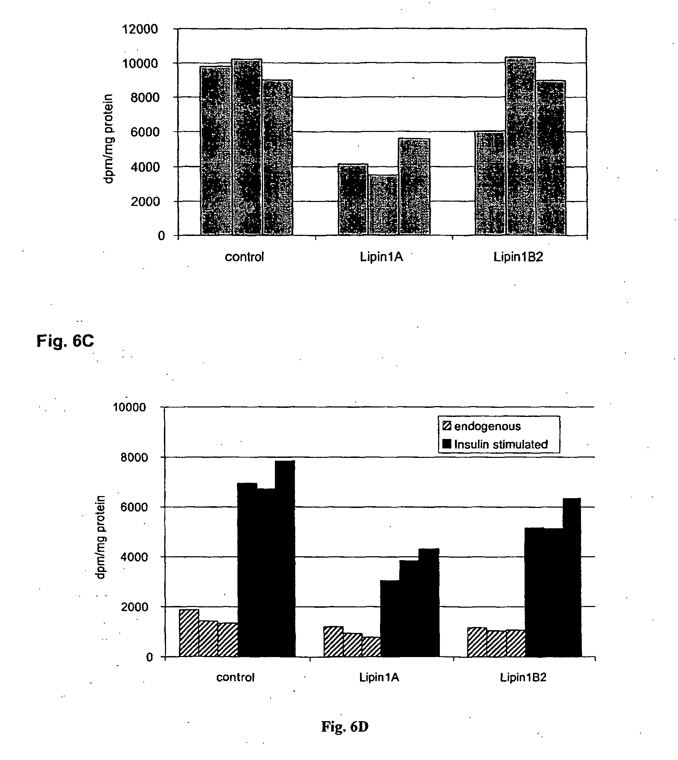

It has also surprisingly been found that overexpression of Lipin1, preferably Lipin1B1 or Lipin1B2, results in a delay in adipocyte differentiation and/or a decrease of phosphorylation of polypeptides that direct PKB-targets or targets further downstream of PKB, like substrates, e.g. direct targets, of mTOR or GSK3. Therefore, in another aspect, the invention provides a method for identifying an activator of lipin function, more preferably Lipin1 function, more preferably Lipin1B and/or Lipin1B2 function, comprising the steps of a) contacting an adipocyte, a preadipocyte, a muscle cell or a pancreas cell, preferably a cell selected from the group consisting of SGBS cells, 293FT cells, 3T3-L1 cells, CCL-136 cells, primary preadipocytes, primary skeletal muscle cells and pancreatic β-cells, most preferably wherein the primary cells are human primary cells, with a test compound, like a small chemical compound or a complex pool of small chemical compounds as, for example, present in combinatorial chemical libraries; and b) detecting a delay in adipocyte differentiation and/or a decrease in phosphorylation of polypeptides that are direct PKB-targets or downstream targets like substrates of mTOR or GSK3. A delay in adipocyte differentiation can, for example, be measured by detecting a decrease in free fatty acid uptake in the cells which have been contacted with the compound to be tested or a decrease in insulin-stimulated lipid synthesis or a decrease in triglyceride levels, as for example by the experiments shown in Figures 6C, 6D or 6E.

### DESCRIPTION OF THE FIGURES

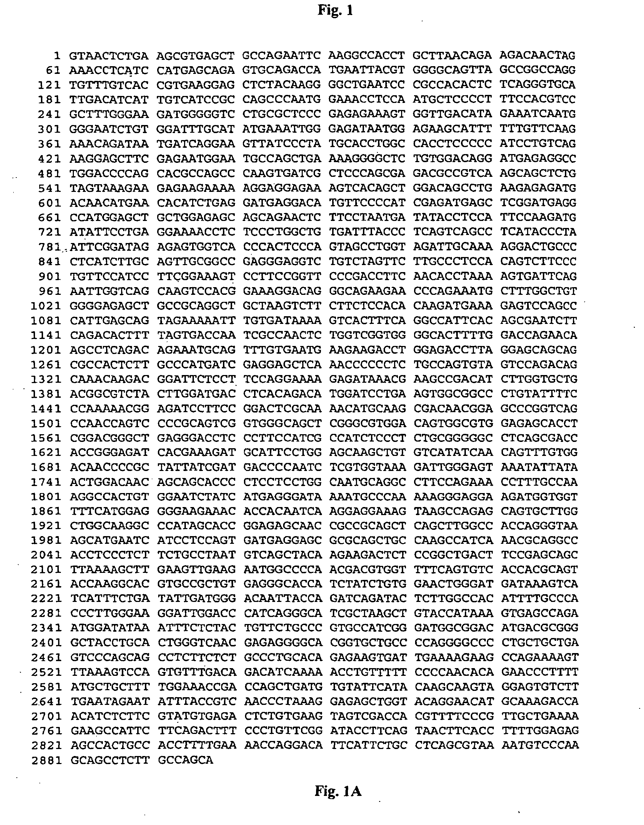

**Figure 1A** shows the nucleic acid sequence of human Lipin1B2 (SEQ ID NO:6).

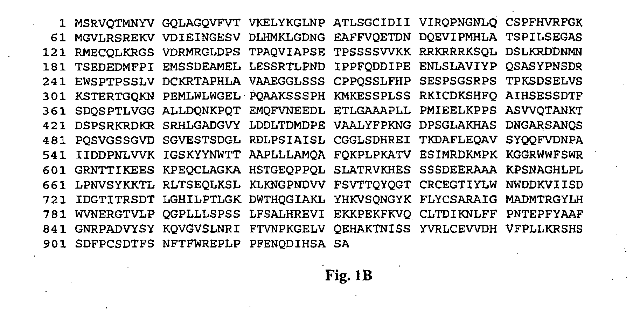

**Figure 1B** shows the amino acid sequence (one-letter code) of human Lipin1B2 (SEQ ID NO:1).

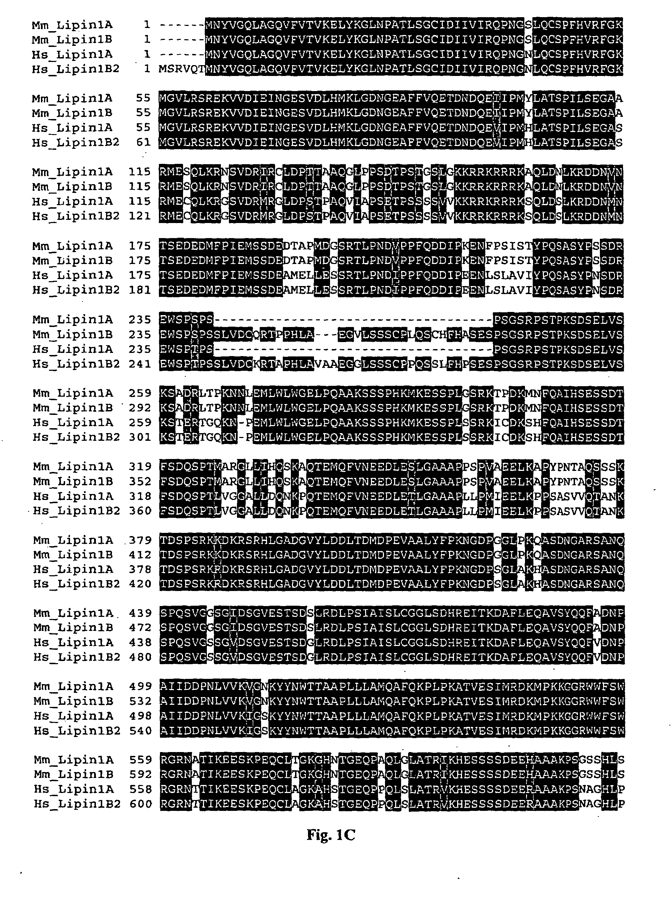

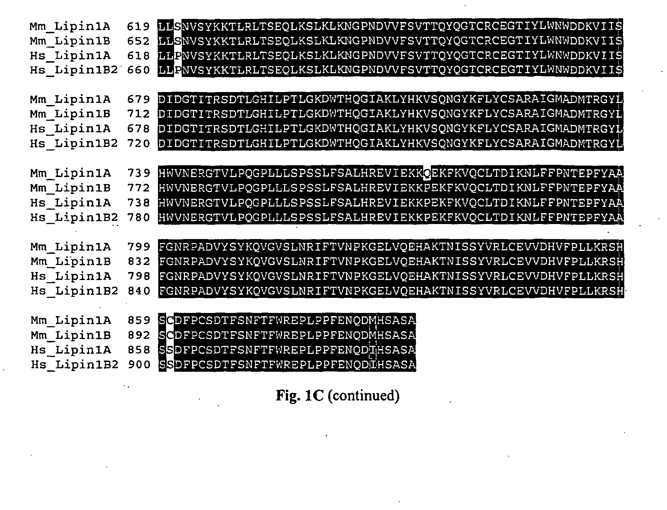

**Figure 1C** shows a multiple sequence alignment of mouse and human Lipin1A and Lipin1B.
**Figure 2** shows the expression of Lipin1A in mammalian (human) tissues.
**Figure 2A** shows the real-time PCR analysis of Lipin1A expression in different human tissues.
**Figure 2B** shows the real-time PCR analysis of Lipin1A expression in subcutaneous adipose tissue in relation to the body mass index (BMI).
**Figure 2C** shows the real-time PCR analysis of Lipin1A expression in a human preadipocyte cell line during the differentiation from preadipocytes to mature adipocytes.
**Figure 3** shows the expression Lipin1B2 in mammalian (human) tissues.
**Figure 3A** shows the real-time PCR analysis of Lipin1B2 expression in different human tissues.
**Figure 3B** shows the real-time PCR analysis of Lipin1B2 expression in subcutaneous adipose tissue in relation to the body mass index (BMI).
**Figure 3C** shows the real-time PCR analysis of Lipin1B2 expression in a human preadipocyte cell line during the differentiation from preadipocytes to mature adipocytes.
**Figure 4** shows the expression of Lipin2 in mammalian (human) tissues.
**Figure 4A** shows the real-time PCR analysis of Lipin2 expression in different human tissues.
**Figure 4B** shows the real-time PCR analysis of Lipin2 expression in subcutaneous adipose tissue in relation to the body mass index (BMI).
**Figure 5** shows the expression of Lipin3 in mammalian (human) tissues.
**Figure 5A** shows the real-time PCR analysis of Lipin3 expression in different human tissues.
**Figure 5B** shows the real-time PCR analysis of Lipin3 expression in subcutaneous adipose tissue in relation to the body mass index (BMI).
**Figure 6** shows *in vitro* assays for the determination of free fatty acid uptake, lipid synthesis, triglyceride levels and marker gene expression in a preadipocyte cell line (SGBS) overexpressing Lipin1A or Lipin1B2.
**Figure 6A** shows the expression of human Lipin1 mRNA in SGBS cells overexpressing Lipin1A or Lipin1B2 and control cells.
**Figure 6B** shows the expression of human Lipin1 proteins in SGBS cells overexpressing Lipin1A or Lipin1B2 and control cells. Shown is the western blot analysis of protein expression. Lanes 1-3: day 0 of differentiation, lanes 4-6: day 7 of differentiation, lanes 7-9: day 12 of differentiation. Lanes 1, 4, and 7 show the expression of Lipin1 and β-actin in control cells, lanes 2, 5, and 8 show the expression of Lipin1 and β-actin in cells overexpressing Lipin1A, lanes 3, 6, and 9 show the expression of Lipin1 and β-actin in cells overespressing Lipin1B2.
**Figure 6C** shows the free fatty acid uptake in SGBS cells overexpressing Lipin1A or Lipin1B2. The Y-axis shows the ³H-oleic acid uptake (radioactivity in dpm/mg protein) and the X-axis the kind of analysed cells: control cells (empty vector), Lipin1A overexpressing cells, and Lipin1B2 overexpressing cells. The ³H-oleic acid uptake is shown for three different sets of samples.
**Figure 6D** shows the lipid synthesis in SGBS cells overexpressing Lipin1A or Lipin1B2. The Y-axis shows the amount of synthesized lipids (shown as dpm per mg protein) and the X-axis shows the controls Lipin1A overexpressing cells, and Lipin1B2 overexpressing cells. Measurements from insulin-stimulated samples are shown as dark grey columns, basic samples are shown as light grey columns. Lipid levels and controls are shown for three different sets of samples.
**Figure 6E** shows the triglyceride levels in SGBS cells overexpressing Lipin1A or Lipin1B2. The Y-axis shows cellular triglyceride levels (shown as µg triglyceride per mg protein) and the X-axis shows days of cell differentiation. Measurements from cells overexpressing Lipin1A are shown as black columns, cells overexpressing Lipin1A are shown as white columns, and control cells (empty vector) are shown as light grey columns. Triglyceride levels and controls are shown for three different sets of samples.
**Figure 6F** shows the expression of human peroxisome proliferative activated receptor, gamma (hPPARγ) in SGBS cells overexpressing Lipin1A or Lipin1B2.
**Figure 6G** shows the expression of human CCAAT/enhancer binding protein (C/EBP), alpha (hCEBPalpha) in SGBS cells overexpressing Lipin1A or Lipin1B2.
**Figure 7** shows *in vitro* assays for the determination of glycogen synthesis and glucose uptake in a Lipin1 loss of function (LOF) preadipocyte cell line (SGBS).
**Figure 7A** shows the expression of Lipin1A mRNA in Lipin1 LOF and control SGBS cells.
**Figure 7B** shows the expression of Lipin1B2 mRNA in Lipin1 LOF and control SGBS cells.
**Figure 7C** shows the expression of human Lipin1 proteins in Lipin1 LOF and control SGBS cells. Shown is the western blot analysis of protein expression. Lanes 1-4: day 0 of differentiation, lanes 5-6: day 7 of adipocyte differentiation, lanes 7-8: day 12 of differentiation. Lane 1 shows the expression of Lipin1 and β-actin in Lipin1A overexpressing cells, lane 2 shows the expression of Lipin1 and β-actin in Lipin1B2 overexpressing cells, lanes 3, 5, and 7 show the expression of Lipin1 and β-actin in control cells, and lanes 4, 6, and 8 show the expression of Lipin1 and β-actin in Lipin1 loss of function cells.
**Figure 7D** shows the glycogen synthesis in Lipin1 LOF SGBS cells. The Y-axis shows the amount of synthesized glycogen (radioactivity in dpm/mg protein) and the X-axis shows controls and Lipin1 LOF SGBS cells. Measurements from insulin-stimulated samples are shown as dark grey columns, basic samples are shown as light grey columns. Glycogen levels and controls are shown for three different sets of samples.
**Figure 7E** shows the glucose uptake in Lipin1 LOF SGBS cells. The Y-axis shows the 2-deoxy-³H-D-glucose uptake (radioactivity in dpm/mg protein) and the X-axis the kind of analysed cells: control cells (empty vector) and Lipin1 LOF SGBS cells. Basal 2-deoxy-³H-D-glucose uptake is shown as light grey columns, insulin stimulated uptake as dark grey columns. The 2-deoxy-³H-D-glucose uptake is shown for three different sets of samples.

### DESCRIPTION OF THE SEQUENCES

SEQ ID NO:1 shows the polypeptide sequence of the human Lipin1B2.
SEQ ID NO:2 shows the nucleotide sequence of the sense strand of the siRNA molecule of the invention.
SEQ ID NO:3 shows the nucleotide sequence of the anti-sense strand of the siRNA molecule of the invention.
SEQ ID NO:4 shows the nucleotide sequence of a DNA sequence complementary to the sense strand of the siRNA molecule of the invention.
SEQ ID NO:5 shows the nucleotide sequence of a DNA sequence complementary to the anti-sense strand of the siRNA molecule of the invention.
SEQ ID NO:6 shows the nucleotide sequence of the human Lipin1B2.
SEQ ID NO:7 shows the nucleotide sequence of a forward primer of the N-terminus of human Lipin1A.
SEQ ID NO:8 shows the nucleotide sequence of a reverse primer of the N-terminus of human Lipin1A.
SEQ ID NO:9 shows the nucleotide sequence of a forward primer of the C-terminus of human Lipin1.
SEQ ID NO:10 shows the nucleotide sequence of a reverse primer of the C-terminus of human Lipin1.
SEQ ID NO:11 shows the nucleotide sequence of a forward primer of the N-terminus of human Lipin1B2.
SEQ ID NO:12 shows the nucleotide sequence of a reverse primer of the N-terminus of human Lipin1B2.
SEQ ID NO:13 shows the nucleotide sequence of a human Lipin1 forward primer.
SEQ ID NO:14 shows the nucleotide sequence of human Lipin1 reverse primer.
SEQ ID NO:15 shows the nucleotide sequence of a human Lipin1 Taqman probe.
SEQ ID NO:16 shows the nucleotide sequence of a human Lipin1A forward primer.
SEQ ID NO:17 shows the nucleotide sequence of a human Lipin1A reverse primer.
SEQ ID NO:18 shows the nucleotide sequence of a Lipin1-specific RNAi sequence.
SEQ ID NO:19 shows the nucleotide sequence of a human Lipin1A Taqman probe.
SEQ ID NO:20 shows the nucleotide sequence of a human Lipin1B2 forward primer.
SEQ ID NO:21 shows the nucleotide sequence of a human Lipin1B2 reverse primer.
SEQ ID NO:22 shows the nucleotide sequence of a human Lipin1B2 Taqman probe.
SEQ ID NO:23 shows the nucleotide sequence of a human Lipin2 forward primer.
SEQ ID NO:24 shows the nucleotide sequence of a human Lipin2 reverse primer.
SEQ ID NO:25 shows the nucleotide sequence of a human Lipin2 Taqman probe.
SEQ ID NO:26 shows the nucleotide sequence of a human Lipin3 forward primer.
SEQ ID NO:27 shows the nucleotide sequence of a human Lipin3 reverse primer.
SEQ ID NO:28 shows the nucleotide sequence of a human Lipin3 Taqman probe.

### EXAMPLES

The following examples are provided to illustrate the invention. They are not to be construed in any way to be limiting to the present invention:

### Example 1: Identification of the human Lipin1B2 gene and protein

The open reading frame of human Lipin1A was cloned in two independent fragments. The sequence encoding the N-terminus of human Lipin was generated by polymerase chain reaction (PCR) (using primers: 5'- CTA GTC TAG A*GA ATT* CCT CGG TGC AGA CCA TGA ATT A -3' (SEQ ID NO:7) and 5'- CCA CTT CAG GAT CCA TGT CTG TG -3' (SEQ ID NO:8)) with human muscle cDNA as template.

The sequence encoding the C-terminal part of human-Lipin was generated by PCR (with primers: 5'- GTC TAC.TTG GAT GAC CTC ACA -3' (SEQ ID NO:9) and 5'- ACC G*CT CGA* GTG CTG GCA AGA GGC TGC TTG G -3' (SEQ ID NO:10)) with human muscle cDNA as template. Both PCR-products were subcloned in pCR-BluntII (Invitrogen) and subsequently joined by digestion with EcoRI and BamHI, and BamHI and Xhol, respectively, and inserting it between the EcoRI and XhoI from the pBluescript vector (Stratagene). The resulting clones were named pTG-hsLipin1A.

Then the Lipin1A cDNA from clone pTG-hsLipin1A was excised by digestion with EcoRI and XhoI and ligating in the EcoRI and XhoI from the Gateway entry vector pENTR1A (Invitrogen). The CMV-based Lipin1A expression vector was created by recombination with the pLenti6/V5-Dest (Invitrogen).

During the cloning procedure a splice variant, Lipin1B2, was identified by PCR amplification on human testis cDNA. Using hsLipin1B2 for 5'- GGG GTA CCG TAA CTC TGA AGC GTG AGC TG -3' (SEQ ID NO:11) as a forward primer and 5'- CCA CTT CAG GAT CCA TGT CTG TG -3' (SEQ ID NO:12) as a reverse primer the N-terminal part containing an additional exon and an earlier start point in comparison to Lipin1A was amplified. The complete open reading frame was cloned as described for Lipin1A.

The Lipin proteins and-nucleic acid molecules coding therefore are obtainable from insect or vertebrate species, e.g. mammals or birds. Particularly preferred are nucleic acids encoding the human Lipin1B2 protein of the invention. The present invention is describing polypeptides comprising the amino acid sequences of the proteins of the invention. Comparisons (Clustal X 1.8 analysis or Clustal W 1.82 analysis, see for example Thompson J. D. *et al.,* (1994) *Nucleic Acids Res.* 22(22):4673-4680; Thompson J. D., (1997) *Nucleic Acids Res.* 25(24):4876-4882; Higgins, D. G. *et al.,* (1996) *Methods Enzymol.* 266:383-402) between the respective proteins of different species (human and mouse) were conducted. Based upon homology, the mouse proteins and each homologous protein or peptide may share at least some activity.

### Example 2: Expression of Lipin polypeptides in human tissues

RNAs isolated from different human tissues were obtained from Biocat, Heidelberg, Germany. Total RNA from Human Adult Normal Adipose (Biocat Order Number R1234003-50); total RNA from Human Adult Normal Stomach (Biocat Order Number R1234248-50); total RNA from Human Adult Normal Small Intestine (Biocat Order Number R1234226-50); total RNA from Human Adult Normal Colon (Biocat Order Number R1234090-50); total RNA from Human Adult Normal Lung, Pancreas, Spleen, Sk. Muscle (Biocat Order Number R8234560); total RNA from Human Adult Normal Heart, Brain, Kidney, Liver (Biocat Order Number R8234559); total RNA from Human Adult Normal Thyroid (Biocat Order Number R1234265-50). RNAs from adipose tissue of donors with known body mass index (BMI) were obtained from Stratech, Soham, UK (L020703; L031703; L051603; L030803; L042903; L061303Abd) and Zen-Bio, NC, USA (L012703).

The RNA was treated with DNase according to the instructions of the manufacturers (for example, from Qiagen, Germany) and as known to those skilled in the art.

Human adipose tissue was obtained from elective surgery. In brief, subcutanous adipose tissue was used to crudely prepare fat pads by liberating from connecting tissue and blood vessels. After collagenase (Collagenase TypI CLS; Biochrom KG; approx. 200 U/ml final conc. in 10% BSA/PBS) digest at 37°C for 90 minutes cells were treated with Erythrocyte lysing buffer (155 mM NH₄Cl, 5.7 mM K₂HPO₄, 0.1 mM EDTA, pH 7.3) at room temperature for 5-10 min. Cells are filtered through nylon-tissue (150µm) and pelleted by centrifugation at 200xg for 10 minutes, resolved in DMEM/HAM F12 (Invitro) and filtered again (BD Falcon cell strainer 70 µm).

For analyzing the role of lipin in the *in vitro* differentiation of mammalian cell culture cells for the conversion of preadipocytes to adipocytes, human SGBS cells were differentiated into mature adipocytes as described by (Hauner *et al.,* (1989) *J* *Clin Invest* 84: 1663-1670). Briefly, cells were grown in DMEM/Nutrient Mix F12, 1 % PenStrep, 17 µM biotin, 33 µM pantothenate, 10% none heat inactivated fetal calf serum. For initiation of differentiation, on day 0 the medium was changed to DMEM/Nutrient Mix F12, 1% Pen/Strep, 17 µM biotin, 33 µM pantothenate, 0,01 mg/ml transferrin, hydrocortisone, 20 nM human insulin, 0,2 nM T3, 25 nM dexamethasone, 250 µM IBMX, 2 µM indomethacine (or 3 µM ciglita-zone). On day 4 of differentiation, the medium was changed to DMEM/Nutrient Mix F12 1%Pen/Strep, 17 µM biotin, 33 µM pantothenate, 0,01 mg/ml transferrin, 100 nM hydrocortisone, 20 nM human insulin, 0,2 nM T3. At various time points of the differentiation procedure, beginning with day 0 (day of confluence and hormone addition), up to 14 days of differentiation, suitable aliquots of cells were taken. RNA was isolated from human cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art.

RNA was isolated from tissues and cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferably using Superscript II RNaseH-Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

Taqman analysis was performed preferably using the following primer/probe pairs:
For the amplification of human Lipin1 sequence:
   Human Lipin1 forward primer (SEQ ID NO:13): 5'- CCG GAG AGC AAC CGC C -3'; human Lipin1 reverse primer (SEQ ID NO:14): 5'- GCT GCG CGC TCC TCA T -3'; human Lipin1 Taqman probe (SEQ ID NO:15): (5/6-FAM)- ACC AGG GTA AAG CAT GAA TCA TCC TCC AGT -(5/6-TAMRA).
For the amplification of human Lipin1A sequence:
   Human Lipin1A forward primer (SEQ ID NO:16): 5'- GGT CAC CCA CTC CCA GTC C -3'; human Lipin1A reverse primer (SEQ ID NO:17): 5'- TGC TGA CCA ATT CTG AAT CAC TTT -3'; human Lipin1A Taqman probe (SEQ ID NO:19): (5/6-FAM)- TCC GGT TCC CGA CCT TCA ACA CC -(5/6-TAMRA).
For the amplification of human Lipin1B2 sequence:
   Human Lipin1B2 forward primer (SEQ ID NO:20): 5'- CCC GGT AGA TTG CAA AAG GA -3'; human Lipin1B2 reverse primer (SEQ ID NO:21): 5'- AGA ACT AGA CAG ACC TCC CTC GG -3'; human Lipin1B2 Taqman probe (SEQ ID NO:22): (5/6-FAM)- TGC CCC TCA TCT TGC AGT TGC G -(5/6-TAMRA).
For the amplification of human Lipin2 sequence:
   Human Lipin2 forward primer (SEQ ID NO:23): 5'- ATG GAA GAC ACT GTC TGT ACC ATA GTG -3'; human Lipin2 reverse primer (SEQ ID NO:24): 5'-CGA GAA GCT CTG CCA CAG ATG -3'; human Lipin2 Taqman probe (SEQ ID NO:25): (5/6-FAM)- CCT GGG TAC ACA GAT GAG CGA CCC A -(5/6-TAMRA).
For the amplification of human Lipin3 sequence:
   Human Lipin3 forward primer (SEQ ID NO:26): 5'- CAC GTG CGT TTT GGC AAG -3'; human Lipin3 reverse primer (SEQ ID NO: 27): 5'- AGC TCA ATG TCT ACC ACC TTC TCC -3'; human Lipin3 Taqman probe (SEQ ID NO:28): (5/6-FAM)- TGG GCG TCC TGC GGT CGC -(5/6-TAMRA).

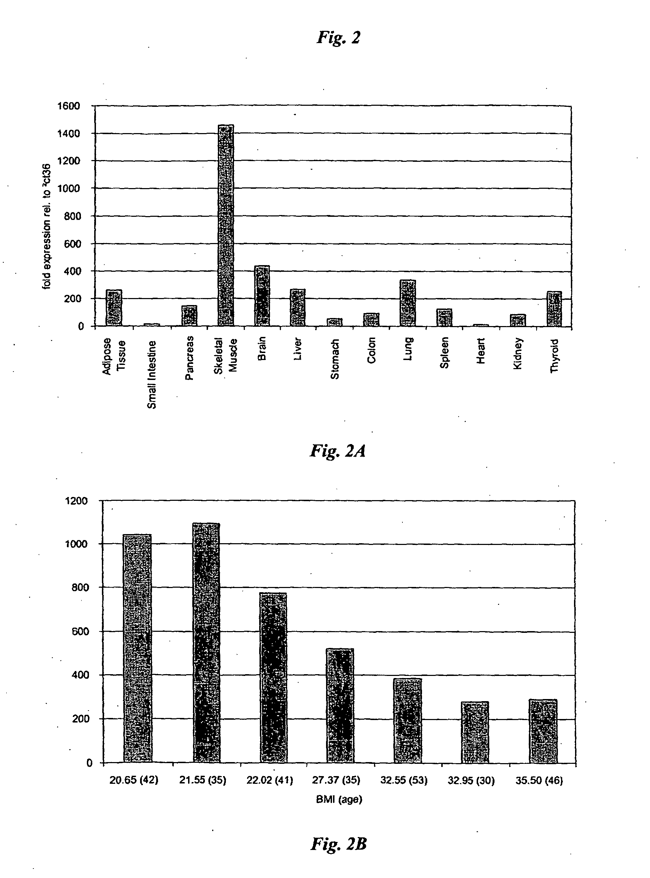

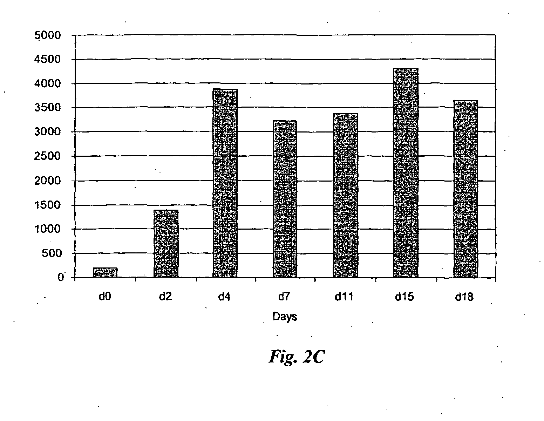

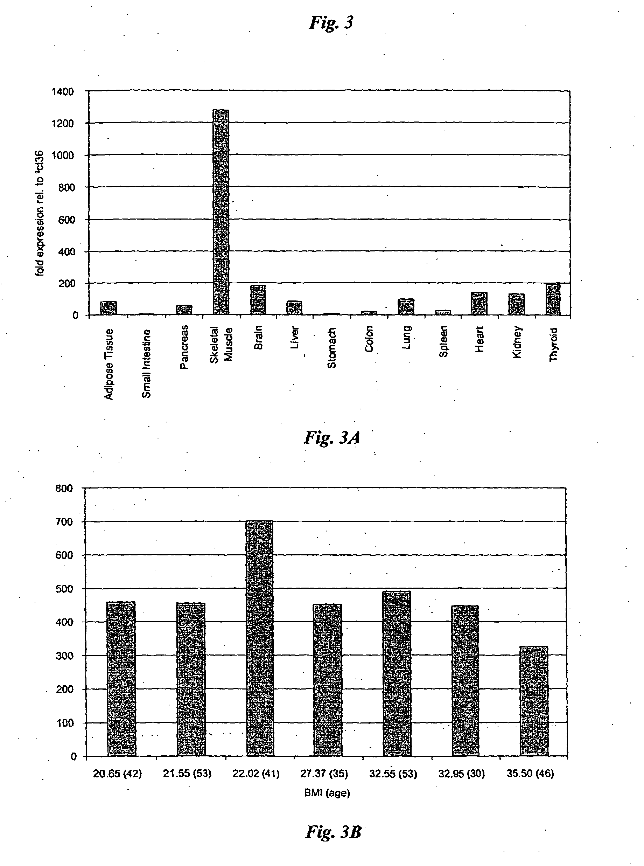

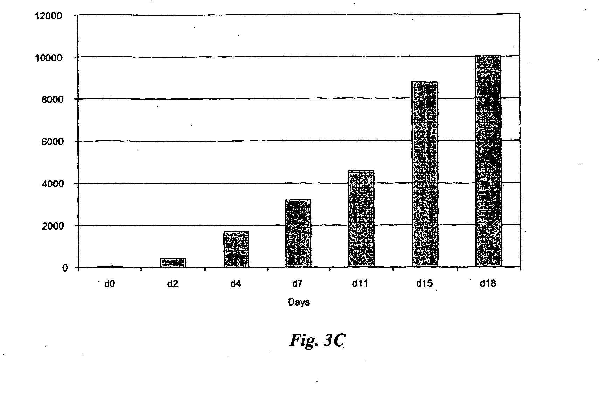

In Figures 2, 3, 4, and 5 the relative RNA-expression is shown on the Y-axis. In Figure 2A, 3A, 4A, and 5A, the tissues tested are given on the X-axis. In Figure 2B, 3B, 4B, and 5B, the body mass index (BMI) and age are given on the X-axis. In Figures 2C and 3C, the X-axis represents the time axis. "d0" refers to day 0 (start of the experiment), "d2"-"d18" refers to day 2-day 18 of adipocyte differentiation.

The function of Lipin1B2 in human metabolism was validated by analyzing the expression of the transcripts in human tissues and by analyzing the role in human adipocyte differentiation.

As shown in Figure 2A, real time PCR (Taqman) analysis of the expression of Lipin1A in human tissues revealed that Lipin1A is expressed in all tissues analysed with highest levels of expression in skeletal muscle, and high expression levels in further tissues, e.g. brain, 'adipose tissue, lung, liver, and thyroid. Furthermore Lipin1A is expressed on lower but still robust levels in pancreas, spleen, kidney, colon, and stomach. In a different human tissue panel, showing the gene expression in a different individual, Lipin1A showed highest expression levels in adipose tissue. The high expression of Lipin1A in metabolic active tissues (adipose tissue and muscle) suggests that this gene plays a role in energy homeostasis.

As shown in Figure 3A, real time PCR (Taqman) analysis of the expression of Lipin1B2 in human tissues revealed that Lipin1B2 is expressed in all tissues analysed with highest levels of expression in skeletal muscle, and high expression levels in further tissues, e.g. brain and thyroid. Furthermore, Lipin1B2 is expressed on lower but still robust levels in adipose tissue, pancreas, liver, lung, heart, and kidney. The high expression of Lipin1B2 in metabolic active tissues (muscle) suggests that this gene plays a role in energy homeostasis.

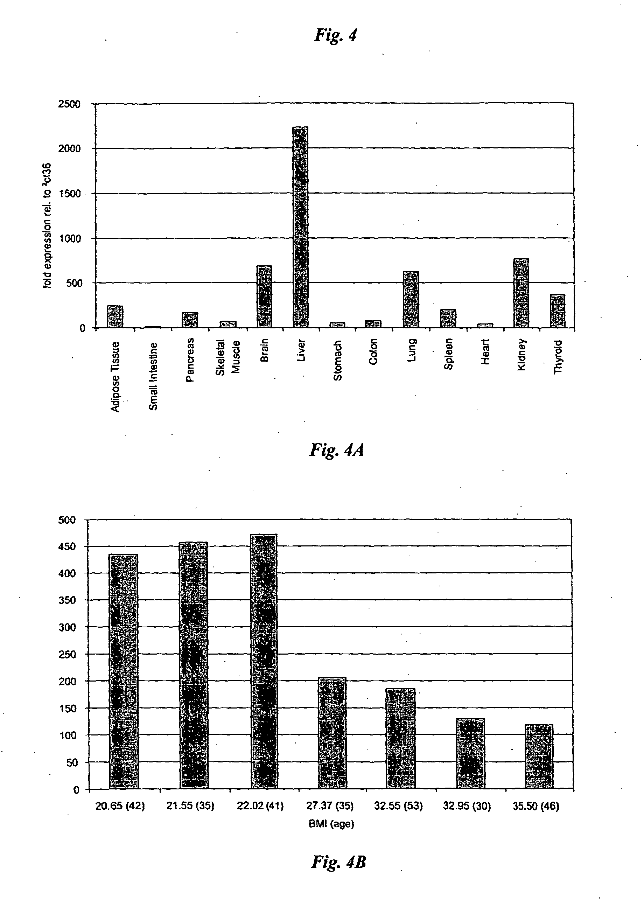

As shown in Figure 4A, real time PCR (Taqman) analysis of the expression of Lipin2 in human tissues revealed that Lipin2 is expressed in all tissues analysed with highest levels of expression in liver, and high expression levels in further tissues, e.g. kidney, brain, lung, adipose tissue, and thyroid. Furthermore, Lipin2 is expressed on lower but still robust levels in spleen, pancreas, skeletal muscle, colon, stomach, and heart.

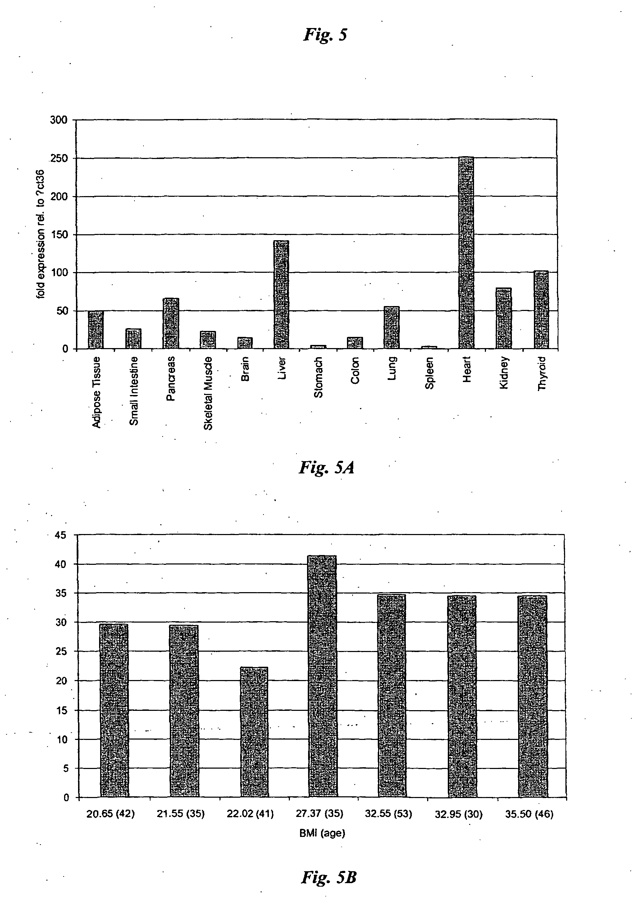

As shown in Figure 5A, real time PCR (Taqman) analysis of the expression of Lipin3 in human tissues revealed that Lipin3 is expressed weakly in all tissues analysed with highest levels of expression in heart, and lower expression levels in further tissues, e.g. liver, thyroid, kidney, adipose tissue, pancreas, and lung. Furthermore, Lipin3 is expressed on lower but still robust levels in small intestine, skeletal muscle, colon, and brain.

As shown in Figure 2B, real time PCR (Taqman) analysis of the expression of Lipin1A in human adipose tissue of female donors with known body mass index (BMI) revealed that Lipin1A expression in adipose tissue correlates with the BMI of the donor, whereas Lipin1B2 expression does not (Figure 2C). Adipose tissues of patients with high BMI display significantly lower Lipin1A mRNA levels than comparable tissues of patients with lower BMI. The age of the individual donors is shown to that this correlation seems to be age-independent.

As shown in Figure 2C, real time PCR (Taqman) analysis of the expression of Lipin1A during *in vitro* adipogenesis of human SGBS cells revealed that Lipin1A is strongly upregulated during adipogenesis. Already early after induction of differentiation (day 2 to day 4) the strong up-regulation is induced and kept on this high level in mature adipocytes (day 11 to day 18). Lipin1B2 is also strongly upregulated during adipogenesis (Figure 3C), but more gradually compared to the regulation of Lipin1A. Since both Lipin1A and Lipin1B2 are strongly upregulated during adipogenesis these genes might play an important role in adipocyte differentiation and function.

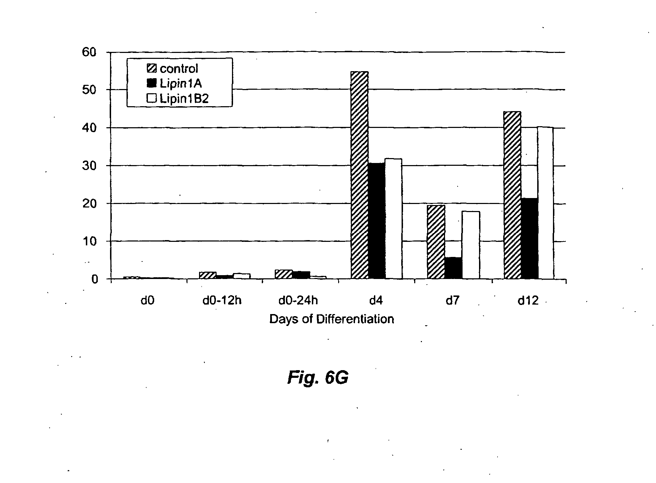

### Example 3: Assays for the determination of free fatty acid uptake, lipid synthesis and triglyceride levels in Lipin1A or Lipin1B2 overexpressing cells (Figure 6)

### Example 3.1.: Lentiviral infection of preadipocytes

Packaging cells were transfected with retroviral plasmids pLenti6/V5-DEST carrying human Lipin1A transgene or the human Lipin1B2 transgene and a selection marker using calcium phosphate procedure. Control cells were infected with pLenti6/V5-DEST carrying no transgene. Briefly, exponentially growing packaging cells were seeded at a density of 2,800,000 cells per T75-flask in 8 ml DMEM + 10 % FCS two days before transfection. 10 min before transfection chloroquine was added directly to the overlying medium (25 µM end concentration). A 2 ml transfection mix consisting of 20 µg plasmid-DNA (candidate:helper-viruses in a 1:1 ratio) and 250 mM CaCl₂ was prepared in a 15 ml plastic tube. The same volume of 2 x HBS (280 µM NaCl, 50 µM HEPES, 1.5 mM Na₂HPO₄, pH 7.06) was added and air bubbles were injected into the mixture for 15 sec. The transfection mix was added drop wise to the packaging cells, distributed and the cells were incubated at 37°C, 5 % CO₂ for 6 hours. The cells were washed with PBS and the medium was exchanged with 8 ml DMEM + 10 % CS per T75-flask. The cells were incubated for 2 days of virus collection at 37°C, 5 % CO₂. The supernatant was then filtered through a 0.45 µm cellulose acetate filter and polybrene (end concentration 8 µg/ml) was added. Human preadipocyte (SGBS) cells in a sub-confluent state were overlaid with the prepared virus containing medium. The infected cells were selected for at least 2 weeks with 5 µg/ml blasticidin. Following selection the cells were checked for transgene expression by quantitative rtPCR (see Figures 6A) and Western blot (see Figure 6B). Over expressing cells were seeded for differentiation.

SGBS cells were maintained as fibroblasts and differentiated into adipocytes as described in the prior art and.supra (see also Hauner *et al.,* 2001). For analysing the role of the proteins disclosed in this invention, *in vitro* assays for the determination of free fatty acid uptake and lipid synthesis were performed.

### Cellular free fatty acid uptake by differentiated adipocytes (Figure 6C)

During the terminal stage of adipogenesis (d13) cells were analysed for their ability to transport long chain fatty acid across the plasma membrane. A modified protocol to the method of Abumrad *et al.* (1991; *Proc Natl Acad Sci USA,* 88:6008-6012) for cellular transportation of fatty acid was established. In summary, cells were washed 3 times with PBS prior to serum starvation. This was followed by incubation in KRBH buffer, supplemented with 0.1% FCS for 2.5 h at 37°C. Uptake of exogenous free fatty acids was initiated by the addition of isotopic media containing non radioactive oleate and (³H)oleate (NEN Life Sciences) complexed to serum albumin in a final activity of 1 µCi/Well/ml in the presence of 5 mM glucose for 30 min at room temperature (RT). For the calculation of passive diffusion (PD) in the absence of active transport (AT) across the plasma membrane 20 mM of phloretin in glucose free media (Sigma) was added for 30 min at room temperature (RT). All assays were performed in duplicate wells. To terminate the active transport 20 mM of phloretin in glucose free media was added to the cells. Cells were lysed in 1 ml 0.1N NaOH and the protein concentration of each well were assessed using the standard Biuret method (Protein assay reagent; Bio-Rad).

We found that active transport of exogenous fatty acids across the plasma membrane of Lipin1A overexpressing cells was significantly lower at day 13 of adipogenesis when compared to control cells, and the active transport of exogenous fatty acids across the plasma membrane of Lipin1B2 overexpressing cells was slightly lower at day 13 of adipogenesis when compared to control cells (Figure 6C). This is in line with the decrease of triglyceride content in these cells (see below).

### Example 3.2.: Lipid synthesis in differentiated adipocytes (Figure 6D)

As shown in Figure 6D, the insulin-stimulated lipid synthesis of SGBS cells over-expressing Lipin1A is significantly decreased during adipogenesis, and the insulin-stimulated lipid synthesis of SGBS cells over-expressing Lipin1B2 is slightly decreased during adipogenesis.

### Example 3.3.: Changes in cellular triglyceride levels during adipogenesis (Figure 6E)

Cell lysates and media were simultaneously analysed in 96-well plates for total protein and triglyceride content using the Bio-Rad DC Protein assay reagent (Bio-Rad, Munich, Germany) according to the manufacturer's instructions and a modified enzymatic triglyceride kit (GPO-Trinder, Sigma) briefly final volumes of reagents were adjusted to the 96-well format as follows: 10 µl sample was incubated with 200 µl reagent A for 5 minutes at 37°C. After determination of glycerol (initial absorbance at 540 nm), 50 µl reagent B was added followed by another incubation for 5 minutes at 37°C (final absorbance at 540 nm). Glycerol and triglyceride concentrations were calculated using a glycerol standard set (Sigma) for the standard curve included in each assay.

As shown in Figure 6E, we found that in Lipin1A overexpressing cells cellular triglyceride levels were decreased on day 7 and day 12 of differentiation when compared to control cells which were transduced with the empty vector. A decrease in triglyceride levels of about 40% in these experiments is significant. We also found that in Lipin1B2 overexpressing cells cellular triglyceride levels were decreased on day 7 and day 12 of differentiation when compared to the control cells. A decrease in triglyceride levels of about 20% in these experiments was observed.

### Example 3.4.: Changes in gene expression

For gene expression analysis cells were washed once with PBS and harvested in 1 ml Trizol (Invitrogen). RNA preparation and taqman analysis was performed as described in Example 2. Gene expression analysis in SGBS cells overexpressing Lipin1A reveals decreased levels of PPAR γamma (Figure 6F), CEBPαlpha (see Figure 6G) and genes downstream of PPAR gamma like aP2 (adipocyte fatty acid binding protein 4), HSL and GLUT4 compared to control cells. In contrast, no significant changes in expression levels of these genes were detected in Lipin1B2 overexpressing cells. These results fit perfectly to the metabolic assays (Figure 6C-6E), where overexpression of Lipin1A resulted in a stronger phenotype than overexpression of the 1B2-form. Apparently, excess of Lipin1A on early time points of the differentiation process leads to suppression/delay of adipogenesis. Therefore, Lipin1 might be an inhibitory factor of adipogenesis when activated/overexpressed on early time points of differentiation. Since, at least by overexpression of Lipin1A, the expression of the very early genes of adipocyte differentiation, PPAR gamma and C/EBP alpha, is affected, Lipin1 might function upstream or in parallel to these genes.

### Example 4: Assays for the determination of glycogen synthesis and glucose uptake in Lipin1 LOF adipocytes (Figure 7)

### Example 4.1.: Loss of function in SGBS adipocytes by RNAi technique

In order to stably inhibit Lipin1 expression, SGBS preadipocytes were engineered by retroviral infection aimed to express a target specific short interfering RNA construct under the control of the human hHl promoter according to Brummelkamp *et al.* (*Science* 2002, Vol 296, p. 550-553). The following Lipin1-specific RNAi sequence (inhibition of both Lipin1 forms) was used: hsLipin-All-I (also referred to as Lipin1): 5'- TGA TTC AGA ATT GGT CAG C - 3' (SEQ ID NO:18).

Other Lipin-specific sequences of potential use for RNAi are

hsLipin All-II: 5' GATCAGGAAGTTATCCCTA 3'; (SEQ ID NO:29).

hsLipin1B-I: 5' TCATCTTGCAGTTGCGGCC 3'; (SEQ ID NO:30).

hsLipin1B-II: 5' GAGGTCTGTCTAGTTCTTG 3' (SEQ ID NO:31).

### Example 4.2.: Lentiviral infection of preadipocytes

Packaging cells were transfected with retroviral plasmids pLenti6/V5-DEST carrying human Lipin1-specific RNAi sequence and a selection marker using calcium phosphate procedure. Control cells were infected with pLenti6/V5-DEST carrying no RNAi sequence. Briefly, exponentially growing packaging cells were seeded at a density of 2,800,000 cells per T75-flask in 8 ml DMEM + 10 % FCS two days before transfection. 10 min before transfection chloroquine was added directly to the overlying medium (25 µM end concentration). A 2 ml transfection mix consisting of 20 µg plasmid-DNA (candidate:helper-viruses in a 1:1 ratio) and 250 mM CaCl₂ was prepared in a 15 ml plastic tube. The same volume of 2 x HBS (280 µM NaCl, 50 µM HEPES, 1.5 mM Na₂HPO₄, pH 7.06) was added and air bubbles were injected into the mixture for 15 sec. The transfection mix was added drop wise to the packaging cells, distributed and the cells were incubated at 37°C, 5 % CO₂ for 6 hours. The cells were washed with PBS and the medium was exchanged with 8 ml DMEM + 10 % CS per T75-flask. The cells were incubated for 2 days of virus collection at 37°C, 5 % CO₂. The supernatant was then filtered through a 0.45 µm cellulose acetate filter and polybrene (end concentration 8 µg/ml) was added. Human preadipocyte (SGBS) cells in a sub-confluent state were overlaid with the prepared virus containing medium. The infected cells were selected for at least 2 weeks with 5 µg/ml blasticidin.

SGBS cells were maintained as fibroblasts and differentiated into adipocytes as described in the prior art and *supra*. The reduction of Lipin1A and Lipin1B2 expression was more than 60% during differentiation as verified by quantitative rtPCR (see Figures 7A and 7B) and western blot (see Figure 7C). For analysing the role of the proteins disclosed in this invention in the *in vitro* assays for the determination of glycogen synthesis and glucose uptake were performed.

### Example 4.3.: Synthesis of glycogen during adipogenesis (Figure 7D)

During adipogenesis cells were analysed for their ability to metabolise lipids. A modified protocol to the method of Jensen *et al.* ((2000) *JBC* 275: 40148) for lipid synthesis was established. Cells were washed 3 times with PBS prior to serum starvation in Krebs-Ringer-Bicarbonate-Hepes buffer (KRBH; 134 nM NaCl, 3.5 mM KCl, 1.2 mM KH₂ PO₄, 0.5 mM MgSO₄, 1.5 mM CaCl₂, 5 mM NaHCO₃, 10 mM Hepes, pH 7.4), supplemented with 0.1% FCS for 2.5h at 37°C. For insulin-stimulated lipid synthesis, cells were incubated with 20 nM human insulin (Sigma; carrier: 0.005 N HCl) for 45 min at 37°C. Basal lipid synthesis was determined with carrier only. ¹⁴C(U)-D-Glucose (NEN Life Sciences) in a final activity of 1 µCi/Well/ml in the presence of 5 mM glucose was added for 30 min at 37°C: For the calculation of background radioactivity, 25 µM Cytochalasin B (Sigma) was used. All assays were performed in triplicate wells. To terminate the reaction, cells were washed 3 times with ice cold PBS, and lysed in 1 ml 0,1 N NaOH. Protein concentration of each well was assessed using the standard Biuret method (Protein assay reagent; Bio-Rad). Total lipids were separated from aqueous phase after overnight extraction in Insta-Fluor scintillation cocktail (Packard Bioscience) followed by scintillation counting.

Our results show that Lipin1 LOF cells were more effective at synthesizing glycogen from exogenous glucose when stimulated with insulin compared to controls (Figure 7D).

### Example 4.5.: Changes in glucose uptake during adipogenesis (Figure 7E)

For the determination of glucose uptake, cells were washed 3 times with PBS prior to serum starvation in Krebs-Ringer-Bicarbonat-Hepes buffer (KRBH; 134 mM NaCl, 3.5 mM KCl, 1.2 mM KH₂ PO₄, 0.5 mM MgSO₄, 1.5 mM CaCl₂, 5 mM NaHCO₃, 10 mM Hepes, pH 7.4), supplemented with 0.1% BSA and 0.5mM Glucose for 2.5h at 37°C. For insulin-stimulated glucose uptake, cells were incubated with 20 nM human insulin (Sigma; carrier: 0.005N HCl) for 45 min at 37°C. Basal glucose uptake was determined with carrier only. Non-metabolizable 2-Deoxy-³H-D-Glucose (NEN Life Science, Boston, USA) in a final activity of 0,4µCi/Well/ml was added for 30 min at 37°C. For the calculation of background radioactivity, 25 µM Cytochalasin B (Sigma) was used. All assays were performed in triplicate wells. To terminate the reaction, cells were washed 3 times with ice cold PBS, and lysed in 320 µl 0.1N NaOH. Protein concentration of each well was assessed using the Bio-Rad DC Protein assay reagent (Bio-Rad), and scintillation counting of cell lysates in 5 ml Ultima-gold cocktail (Packard Bioscience, Groningen, Netherlands) was performed.

As shown in Figure 7E the insulin stimulated glucose uptake of Lipin1 LOF SGBS cells is increased by more than 25% during adipogenesis. This increase in glucose and therefore energy uptake of the cells is most likely the reason for the increased glycogen uptake we observed during SGBS differentiation (see Figure 7D).

## Claims

1. An siRNA molecule or shRNA molecule comprising the double-stranded region

5' UGAUUCAGAAUUGGUCAGC 3' (SEQ ID NO:2)

3' ACUAAGUCUUAACCAGUCG 5' (SEQ ID NO:3).

2. The siRNA molecule or shRNA molecule of claim 1, wherein a first strand having the nucleotide sequence SEQ ID NO:2 and 2 or 3 nucleotides added to the 3' end of SEQ ID NO:2 is paired to a second strand having the nucleotide sequence SEQ ID NO:3 and 3 or 4 nucleotides added to the 3' end of SEQ ID NO:3, preferably wherein the added nucleotides are U residues.

3. A polypeptide comprising the amino acid sequence of SEQ ID NO:1.

4. A nucleic acid comprising a nucleotide sequence encoding a polypeptide according to claim 3.

5. The nucleic acid of claim 4, wherein the nucleic acid is DNA or RNA.

6. A recombinant expression cassette comprising
a) the nucleic acid according to claims 4 or 5 operatively linked to at least one regulatory sequence for expression of the polypeptide of claim 3; or
b) a nucleic acid sequence SEQ ID NO:4 and a nucleic acid sequence SEQ ID NO:5, operatively linked to at least one regulatory sequence for transcription of an RNA comprising the nucleotide sequences SEQ ID NO:2 and SEQ ID NO:3; or
c) a nucleic acid sequence SEQ ID NO:4 operatively linked to at least one first regulatory sequence for transcription of an RNA comprising the nucleotide sequence SEQ ID NO:2 in combination with a nucleic acid sequence SEQ ID NO:5, operatively linked to at least one second regulatory sequence for transcription of an RNA comprising the nucleotide sequence SEQ ID NO:3.

7. A combination of two expression cassettes, wherein a first expression vector comprises a nucleic acid sequence SEQ ID NO:4, operatively linked to at least one regulatory sequence, for transcription of an RNA comprising the nucleotide sequence SEQ ID NO:2, and wherein a second expression vector comprises a nucleic acid sequence SEQ ID NO:5, operatively linked to at least one regulatory sequence, for transcription of an RNA comprising the nucleotide sequence SEQ ID NO:3.

8. A host cell comprising an siRNA or shRNA according to claims 1 or 2 or an expression cassette according to claim 6 or a combination of two expression cassettes according to claim 7.

9. The host cell of claim 8, wherein the host cell is a mammalian cell, preferably a human cell, a mouse cell or a rat cell, more preferably an adipocyte, a preadipocyte, a muscle cell or a pancreas cell, even more preferably a cell selected from the group consisting of SGBS cells, 293FT cells, 3T3-L1 cells, CCL-136 cells, primary preadipocytes, primary skeletal muscle cells and pancreatic β-cells, most preferably wherein the cells are human cells.

10. A polypeptide according to claim 3 or an siRNA or shRNA according to claims 1 or 2 or an expression cassette according to claim 6 or a combination of two expression cassettes according to claim 7 for use in medicine.

11. A pharmaceutical composition comprising a polypeptide according to claim 3 or an siRNA or shRNA according to claims 1 or 2 or an expression cassette according to claim 6 or two expression cassettes according to claim 7 and a pharmaceutically acceptable carrier.

12. The pharmaceutical composition according to claim 11, further comprising an insulin sensitizer or insulin secretagogue, in particular further comprising a thiazolidinedione, an insulin secretagogue, a biguanide or an alpha glucosidase inhibitor.

13. Use of
an siRNA or shRNA according to claims 1 or 2 or an expression cassette according to claim 6 b) or two expression cassettes according to claim 7 or another inhibitor of lipin function, particularly of Lipin1 function, or use of a pharmaceutical composition according to one of claims 11 or 12 in the manufacture of a pharmaceutical for the prophylaxis and/or treatment and/or for delaying the onset of a disorder or a disease selected from the group consisting of diabetes mellitus, arteriosclerosis, muscular dystrophy, heart attack and stroke.

14. Use of an activator of lipin function, particularly of Lipin1 function, for the prophylaxis and/or treatment and/or for delaying the onset of a disorder or a disease selected from the group consisting of obesity and cancer.

15. Use of claims 13 and 14, wherein the medicament is to be administered to a mammal, preferably wherein said mammal is a human being, a cat, a dog, a mouse or a rat.

16. Use of an siRNA or shRNA directed against a nucleotide sequence on the Lipin1-mRNA, preferably the Lipin-1A- or Lipin-1B1- or Lipin1B2-mRNA, either within 50 nucleotides (nt) upstream of the sequence 5' UGAUUCAGAAUUGGUCAGC 3' starting from the 5' U or 50 nt downstream of said sequence starting from the 3' C, preferably within 30 nt upstream or 30 nt downstream of said sequence, more preferably within 20 nt upstream or 20 nt downstream of said sequence, most preferably within 15 nt upstream or 15 nt downstream of said sequence, for reducing expression of Lipin1, preferably Lipin1A or Lipin1B1, or Lipin1B2, ex *vivo*, preferably *in vitro.*

17. A method of producing an siRNA or shRNA capable of reducing expression of Lipin1, preferably Lipin1B1 or Lipin1B2, comprising the steps of
a) providing an siRNA or shRNA directed against a nucleotide sequence on the Lipin1-mRNA, preferably the Lipin1B1- or Lipin1B2-mRNA, either within 50 nucleotides (nt) upstream of the sequence 5' UGAUUCAGAAUUGGUCAGC 3' starting from the 5' U or 50 nt downstream of said sequence starting from the 3' C, preferably within 30 nt upstream or 30 nt downstream of said sequence, more preferably within 20 nt upstream or 20 nt downstream of said sequence, most preferably within 15 nt upstream or 15 nt downstream of said sequence; and
b) optionally testing whether said siRNA or shRNA reduces expression of Lipin1, preferably Lipin1B1 or Lipin1B2.

18. A method for identifying an inhibitor of Lipin1 function, preferably Lipin1B1 or Lipin1B2 function, comprising the steps of
a) contacting an adipocyte, a preadipocyte, a muscle cell or a pancreas cell, preferably a cell selected from the group consisting of SGBS cells, 293FT cells, 3T3-L1 cells, CCL-136 cells, primary preadipocytes, primary skeletal muscle cells and pancreatic β-cells, more preferably wherein the primary cells are human primary cells, with a test compound; and
b) detecting an increase in glucose uptake, glycogen formation or an increase in phosphorylation of polypeptides that are direct PKB-targets or downstream targets like substrates of mTOR or GSK3.

19. A method for identifying an activator of Lipin1 function, preferably Lipin1B1 or Lipin1B2 function, comprising the steps of
a) contacting an adipocyte, a preadipocyte, a muscle cell or a pancreas cell, preferably a cell selected from the group consisting of SGBS cells, 293FT cells, 3T3-L1 cells, CCL-136 cells, primary preadipocytes, primary skeletal muscle cells and pancreatic β-cells, more preferably wherein the primary cells are human primary cells, with a test compound; and
b) detecting a delay in adipocyte differentiation or a decrease in phosphorylation of polypeptides that are direct PKB-targets or downstream targets like substrates of mTOR or GSK3.
